# EUROPEAN PATENT SPECIFICATION

(11) **EP 0 432 648 B1**
(45) Date of publication and mention of the grant of the patent: **02.08.1995**
(21) Application number: 90123474.0
(22) Date of filing: 06.12.1990
(51) Int. Cl.: C07D 209/40, A61K 31/40

(54) **Isatine derivatives, their preparation and use**
Isatinderivate, ihre Herstellung und Verwendung
Dérivés de l'isatine, leur préparation et utilisation

(30) Priority: 11.12.1989 DK 6248/89; 19.12.1989 DK 6470/89; 12.01.1990 DK 85/90; 12.01.1990 DK 86/90; 12.02.1990 DK 363/90; 31.08.1990 DK 2093/90
(43) Date of publication of application: 19.06.1991
(73) Proprietor: NEUROSEARCH A/S, DK-2600 Glostrup (DK)
(72) Inventor: Wätjen, Frank, DK-3500 Vaerlose (DK); Drejer, Jorgen, DK-3500 Vaerlose (DK); Jensen, Leif Helth, DK-1573 Kobenhavn V. (DK)
(74) Representative: Grünecker, Kinkeldey, Stockmair & Schwanhäusser Anwaltssozietät

(56) References cited:
- EP-A- 0 010 398
- Chemical Abstracts, vol. 103, no. 3, 22nd July 1985, page 17, column 1, ref.-no. 16405s, Columbus, Ohio, USA; Cedere D. et al.: "Reversible inhibitors of monoamine oxidase in the indolinone series" page 17; column 1; ref.no. 16405S & Khim.-Farm.Zh.1984 vol.18,no.5,pages 555-8
- Wissenschaftliche Zeitschrift der Ernst-Moritz-Arndt-Universität Greifswald, Mathematisch-Naturwissenschaftliche Reihe, vol. XXXV, no. 4, 1986, DDR, pages 39-44; Baehnisch S. et al.: "Zur Wirkung substituierter Indoline am isolierten Rattenfundusstreifen"
- Die Pharmazie, vol. 39, no. 10, page 713, October 1984, DDR; Fischer W. et al.:"Indoline und serotonerge Effekte an isolierten Organpräparaten"
- Die Pharmazie, vol. 37, no. 12, pages 858-861, December 1982, DDR ; Fischer W. et al.: "Antikonvulsive Wirkungen von Indolinderivaten"

## Description

The present invention relates to the use of compounds having excitatory amino acid antagonizing properties for preparing a medicament, pharmaceutical compositions comprising such compounds, and to novel compounds having excitatory amino acid antagonizing properties and to the preparation of such compounds.

It is well known from
Wiss, Z. Ernst-Moritz-Arndt-Univ. Greifswald, Math.-nat.wiss.
Reihe 35, 39-44 (1986) 4,
Pharmazie 39, H.10, 713 (1984),
Pharmazie 37, H.12, 858-861 (1982),
Neuroscience Letters 107, 327-330 (1989),
PCT patent application International Publication Number WO 89/03818, and
Khim.-Farm.zh. 23(11), 1349-1352 (1989),
that certain of the chemical entities comprised within the scope of method of treatment according to the present invention are known to possess biological activity.

Chemical Abstracts vol. 103 (1985), abstract No. 16405s discloses indolinone derivatives which can function as reversible inhibitors of monoamine oxidase; some of the compounds showed low toxicity. An excitatory amino acid antagonizing property is not known from said compounds.

EP-A-0010398 discloses isatin derivatives which are useful for treating allergic symptoms. And excitatory amino acid antagonizing property of said compound is not disclosed.

It is an object of the present invention to provide novel compounds useful for the treatment of diseases in mammals, including a human, acting by antagonizing an excitatory amino acid in such mammal. Said object is achieved by compounds having the formula 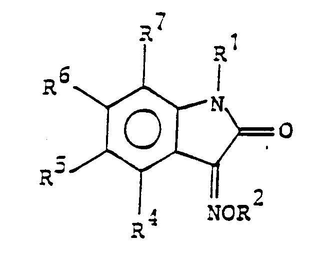
wherein R¹ is hydrogen, C₁₋₆-alkyl which may be branched, C₃-₇-cyclo alkyl, benzyl, acyl, hydroxy, C₁₋₆-alkoxy, CH₂CO₂R' wherein R' is hydrogen or C₁₋₆-alkyl which may be branched, CH₂CN, CH₂CONR^{IV}R^{V} where-in R^{IV} and R^{V} independently are hydrogen or C₁₋₆-alkyl, or CH₂C(=NOH)NH₂;
R² is hydrogen, benzyl, C₁₋₆-alkyl which may be branched, or C₃₋₇-cycloalkyl;
R⁵ is NO₂, F, CF₃, SO₂NR''R''' wherein R'' and R''' independently are hydrogen or C₁₋₆-alkyl, or CN; and
R⁴, R⁶, R⁷ independently are hydrogen, C₁₋₆-alkyl which may be branched, phenyl, halogen, C₁₋₆-alkoxy, NO₂, CN, SO₂NR''R''' wherein R'' and R''' independently are hydrogen or C₁₋₆-alkyl, or CF₃, or R⁶ and R⁷ together form an additional 4 to 7 membered ring which may be aromatic or partial saturated and which may be substituted with halogen, NO₂, CF₃, CN, SO₂NR''R''' wherein R'' and R''' independently are hydrogen or C₁₋₆-alkyl, and R⁴ has the meaning set forth above, with the proviso that R⁵ is different from NO₂ and F when R¹, R⁴, R⁶ and R⁷ are hydrogen and R² is nydrogen or benzyl; and that R⁵ is different from NO₂ when R¹, R², R⁴ and R⁶ are hydrogen and R⁷ is NO₂.

The further object of the present invention is to provide novel pharmaceutical compositions useful for the treatment of diseases in mammals, including a human, acting by antagonizing an excitatory amino acid in such mammal.

Said object is achieved by a pharmaceutical composition comprising an effective excitatory amino acid antagonizing amount of a compound having the formula 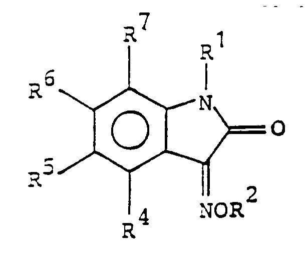
wherein R¹ is hydrogen, C₁₋₆-alkyl which may be branched, C₃₋₇-cyclo alkyl, benzyl, acyl, hydroxy, C₁₋₆-alkoxy, CH₂CO₂R' wherein R' is hydrogen or C₁₋₆-alkyl which may be branched, CH₂CN, CH₂CONR^{IV}R^{V} wherein R^{IV} and R^{V} independently are hydrogen or C₁₋₆-alkyl, or CH₂C(=NOH)NH₂;
R² is hydrogen, benzyl, C₁₋₆-alkyl which may be branched, or C₃₋₇-cycloalkyl;
R⁵ is NO₂, F, CF₃, SO₂NR''R''' wherein R'' and R''' independently are hydrogen or C₁₋₆-alkyl, or CN; and
R⁴, R⁶, R⁷ independently are hydrogen, C₁₋₆-alkyl which may be branched, phenyl, halogen, C₁₋₆-alkoxy, NO₂, CN, SO₂NR''R''' wherein R'' and R''' independently are hydrogen or C₁₋₆-alkyl, or CF₃, or R⁶ and R⁷ together form an additional 4 to 7 membered ring which may be aromatic or partial saturated and which may be substituted with halogen, NO₂, CF₃, CN, SO₂NR''R''' wherein R'' and R''' independently are hydrogen or C₁₋₆-alkyl, and R⁴ has the meaning set forth above.

A further object of the present invention is to provide compounds which are useful for preparing a medicament for treating conditions in mammals, including a human, sensitive to an excitatory amino acid.

Said object is achieved by using an indole-2,3-dione-3-oxime compound having the formula 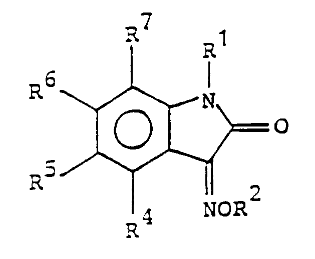
wherein
R¹ is hydrogen, C₁₋₆-alkyl which may be branched, C₃₋₇-cycloalkyl, benzyl, phenyl
acyl, hydroxy, C₁₋₆-alkoxy, CH₂CO₂R' wherein R' is hydrogen or C₁₋₆-alkyl which may be branched, CH₂CN,
CH₂CONR^{IV}R^{V} wherein R^{IV} and R^{V} independently are hydrogen or C₁₋₆-alkyl, or CH₂C(=NOH)NH₂;
R² is hydrogen, benzyl, C₁₋₆-alkyl which may be branched, or C₃₋₇-cycloalkyl;
R⁵ is NO₂, F, CF₃, SO₂NR''R''' wherein R'' and R''' independently are hydrogen or C₁₋₆-alkyl, or CN; and R⁴, R⁶, R⁷ independently are hydrogen, C₁₋₆-alkyl which may be branched, phenyl, halogen, C₁₋₆-alkoxy, NO₂, CN, SO₂NR''R''' wherein R'' and R''' independently are hydrogen or C₁₋₆-alkyl, or CF₃, or R⁶ and R⁷ together form an additional 4 to 7 membered ring which may be aromatic or partial saturated and which may be substituted with halogen, NO₂, CF₃, CN, SO₂NR''R''' wherein R'' and R''' independently are hydrogen or C₁₋₆-alkyl, and R⁴ has the meaning set forth above, for the preparation of a medicament useful in the treatment of conditions sensitive to an excitatory amino acid.

In a preferred embodiment a compound is used wherein at least one of R⁴, R⁵, R⁶ and R⁷ is NO₂, CF₃, CN, SO₂NR''R''', or halogen and R¹, R², R⁴, R⁵, R⁶, R⁷, R'', and R''' otherwise have the meanings set forth in claim 1.

In a further preferred embodiment a compound is used wherein R⁵ is NO₂, F, CF₃, or CN.

In a method of antagonizing the biological effects of an excitatory amino acid the compound is administered in the form of a pharmaceutical composition thereof, in which it is present together with a pharmaceutically acceptable carrier or diluent.

Preferred compounds have the formula 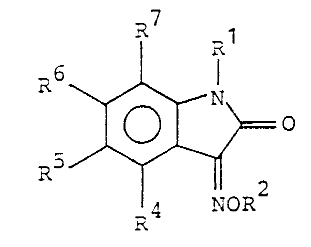
wherein R¹ is C₁₋₆-alkyl which may be branched, C₃₋₇-cycloalkyl, benzyl, acyl, hydroxy, C₁₋₆-alkoxy, CH₂CO₂R' wherein R' is hydrogen or C₁₋₆-alkyl which may be branched, CH₂CN, CH₂CONR^{IV}R^{V} wherein R^{IV} and R^{V} independently are hydrogen or C₁₋₆-alkyl, or CH₂C(=NOH)NH₂;
R² is hydrogen, benzyl, C₁₋₆-alkyl which may be branched, or C₃₋₇-cycloalkyl;
R⁵ is NO₂, F, CF₃, SO₂NR''R''' wherein R'' and R''' independently are hydrogen or C₁₋₆-alkyl, or CN; and
R⁴, R⁶, R⁷ independently are hydrogen, C₁₋₆-alkyl which may be branched, phenyl, halogen, C₁₋₆-alkoxy, NO₂, CN, SO₂NR''R''' wherein R'' and R''' independently are hydrogen or C₁₋₆-alkyl, or CF₃, or R⁶ and R⁷ together form an additional 4 to 7 membered ring which may be aromatic or partial saturated and which may be substituted with halogen, NO₂, CF₃, CN, SO₂NR''R''' wherein R'' and R''' independently are hydrogen or C₁₋₆-alkyl, and R⁴ has the meaning set forth above, Further preferred compounds have the formula 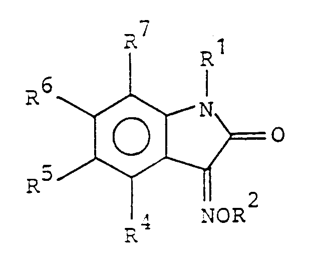
wherein
R¹ is hydrogen,
R² is hydrogen, benzyl, C₁₋₆-alkyl which may be branched, or C₃₋₇-cycloalkyl;
R⁵ is NO₂, F, CF₃, SO₂NR''R''' wherein R'' and R''' independently are hydrogen or C₁₋₆-alkyl, or CN; and
R⁴, R⁶, R⁷ independently are hydrogen, C₁₋₆-alkyl which may be branched, phenyl, halogen, C₁₋₆-alkoxy, NO₂, CN, SO₂NR''R''' wherein R'' and R''' independently are hydrogen or C₁₋₆-alkyl, or CF₃, or R⁶ and R⁷ together form an additional 4 to 7 membered ring which may be aromatic or partial saturated and which may be substituted with halogen, NO₂, CF₃, CN,
SO₂NR''R''' wherein R'' and R''' independently are hydrogen or C₁₋₆-alkyl, and R⁴ has the meaning set forth above; and that R⁵ is different from NO₂ and F when R¹, R⁴, R⁶ and R⁷ are hydrogen and R² is hydrogen or benzyl; and that R⁵ is different from NO₂ when R¹, R², R⁴ and R⁶ are hydrogen and R⁷ is NO₂.

In a further preferred compound R⁴ and R⁵ independently are hydrogen, F, NO₂, CN, CF₃, or SO₂NR''R''' wherein R'' and R''' independently are hydrogen or C₁₋₆-alkyl, and wherein R⁶ and R⁷ together form an additional 4 to 7 membered ring which may be aromatic or partial saturated and which may be substituted with halogen, NO₂, CF₃, CN, SO₂NR''R''' wherein R'' and R''' independently are hydrogen or C₁₋₆-alkyl.

In a further preferred compound as above the additional ring formed by R⁶ and R⁷ is substituted with halogen, NO₂, CF₃, CN or SO₂NR''R''' wherein R'' and R''' independently are hydrogen or C₁₋₆-alkyl.

Particularly preferred compounds are 5-nitro-1H-benz[g]indole-2,3-dione-3-oxime;
5,7-dinitro-1-methyl-1H-indole-2,3-dione-3-(0-methyloxime);
and 5-nitro-1H-6,7,8,9-tetrahydro-benz[g]indole-2,3-dione -3-oxime.

Further it is provided a method of preparing a compound having the formula 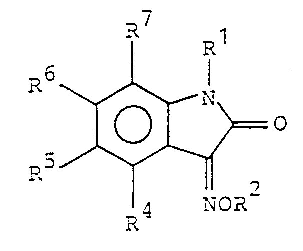
wherein R¹ is hydrogen, C₁₋₆-alkyl which may be branched, C₃₋₇-cycloalkyl, benzyl, acyl,
hydroxy, C₁₋₆-alkoxy, CH₂CO₂R' wherein R' is hydrogen or C₁₋₆-alkyl which may be branched, CH₂CN, CH₂CONR^{IV}R^{V} wherein R^{IV} and R^{V} independently are hydrogen or C₁₋₆-alkyl, or CH₂C(=NOH)NH₂; R² is hydrogen, benzyl, C₁₋₆-alkyl which may be branched, or C₃₋₇-cycloalkyl;
R⁵ is NO₂, F, CF₃, SO₂NR''R''' wherein R'' and R''' independently are hydrogen or C₁₋₆-alkyl, or CN; and
R⁴, R⁶, R⁷ independently are hydrogen, C₁₋₆-alkyl which may be branched, phenyl, halogen, C₁₋₆-alkoxy, NO₂, SO₂NR''R''' wherein R'' and R''' independently are hydrogen or C₁₋₆-alkyl, CN, or CF₃, or R⁶ and R⁷ together form an additional 4 to 7 membered ring which may be aromatic or partial saturated and which may be substituted with halogen, NO₂, CF₃, CN, SO₂NR''R''' wherein R'' and R''' independently are hydrogen or C₁₋₆-alkyl, and R⁴ has the meaning set forth above; and that R⁵ is different from NO₂ and F when R¹, R⁴, R⁶ and R⁷ are hydrogen and R² is hydrogen or benzyl; and that R⁵ is different from NO₂ when R¹, R² R⁴ and R⁶ are hydrogen and R⁷ is NO₂, comprising the step of reacting a compound of the formula 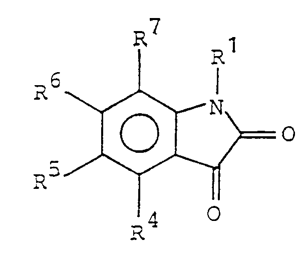
wherein R¹, R⁴, R⁵, R⁶ and R⁷ have the meanings set forth above, with a compound having the formula NH₂OR², wherein R² has the meaning set forth above.

In preferred compounds used for preparing the medicament at least one of R⁴, R⁵, R⁶ or R⁷ is an electron withdrawing substituent such as NO₂, CF₃, CN, SO₂NR''R''', or halogen and R¹, R², R⁴, R⁵, R⁶, R⁷, R'' and R''' otherwise have the meanings set forth above.

Preferred compounds to be employed according to the invention and preferred compounds according to the invention per se are for example:
5,7-dinitro-1-methyl-1H-indole-2,3-dione-3-(O-methyloxime)
5-bromo-7-nitro-1H-indole-2,3-dione-3-oxime,
5,7-dinitro-1-methyl-1H-indole-2,3-dione-3-oxime,
5-nitro-1H-benz[g]indole-2,3-dione-3-oxime,
5-nitro-1H-6,7,8,9-tetrahydro-benz[g]indole-2,3-dione-3-oxime.

### Biological Activity

The compounds of the invention exhibit valuable biological properties because of their strong excitatory amino acid (EAA) (glycine, glutamate, quisqualate, ATPA, AMPA, kainate, NMDA) antagonizing properties.

For example compounds of the invention exhibit strong pharmacological in vivo ATPA and quisqualate antagonizing effects demonstrating their utility as novel orally-bioavailable excitatory amino acid antagonists, which makes them useful in the treatment of for example excitatory amino acid dependent psychosis, excitatory amino acid dependent anoxia, excitatory amino acid dependent ischemia, excitatory amino acid dependent convulsions, and excitatory amino acid dependent migraine.

Compounds of the invention will inhibit ATPA-induced rigidity and quisqualate or NMDA-induced convulsions with an ED₅₀ in the range of 0.1-10.0 mg/kg. Examples of such compounds are 5,7-dinitro-1-methyl-1H-indole-2,3-dione-3-(O-methyloxime) and 5,7-dinitro-1-(ethoxy carbonyl methyl)-1H-indole-2,3-dione-3-(0-methyloxime).

Compounds of the invention show potent in vitro affinity for the glutamate subreceptors kainate, quisqualate and glycine receptors. These properties make the compounds useful in the treatment of human malfunctions related to the excitatory amino acids (EAA).

For example some compounds of the invention exhibit binding at the ³H-kainate, ³H-AMPA and/or ³H-glycine binding sites with IC₅₀ in the range of 10-100 »M. Examples of such compounds are for example
5-bromo-7-nitro-1H-indole-2,3-dione-3-oxime,
5-nitro-1H-indole-2,3-dione-3-oxime,
5,7-dinitro-1H-indole-2,3-dione-3-oxime,
5,7-dinitro-1-methyl-1H-indole-2,3-dione-3-oxime,
1H-benz[g]indole-2,3-dione-3-oxime,
5-nitro-1H-benz[g]indole-2,3-dione-3-oxime, and 5-nitro-1H-6,7,8,9-tetrahydro-benz[g]indole-2,3-dione-3-oxime.

Also the compounds of the invention as a secondary result of their EAA-antagonizing properties have been found to antagonize cocaine-induced hypermotility. For example the most potent compounds of the invention have been found to have an ED₅₀ in the range of 0.5-1.0 mg/kg in this test when administered orally. An example of such a compound is 5,7-dinitro-1-methyl-1H-indole-2,3-dione-3-(O-methyloxime).

Furthermore it has been found that some of the compounds of the invention are metabolites of other compounds of the invention and that the metabolites exhibit biological activity in the same range or are even more potent than the precursor compounds or the invention. Accordingly both such parent or precursor compounds and such metabolites fall within the scope or the invention. Some of the compounds named in the foregoing will be recognized as metabolites of precursor compounds named in the foregoing, and vise versa.

Such metabolites are for example:
N-dealkylated derivatives, 1-N-hydroxyalkyl derivatives, 1-N-hydroxy derivatives, 1-N-oxide derivatives, O-dealkylated derivatives, pyrrolo ring opened hydrolyzed derivatives, pyrrolo ring opened hydrolyzed and decarboxylated derivatives as well as combinations of such metabolisation reactions.

### Biological testing

The above mentioned tests are performed as described in more detail below and are based upon the principles also described hereinafter.

### ATPA-induced rigidity

The selective quisqualate receptor agonist ATPA induces rigidity in female NMRI mice at doses between 3 and 15 mg/kg and clonic-tonic seizures and death, probably due to respiratory arrest, at doses between 15 and 40 mg/kg after intravenous (i.v.) administration.

### Method

ATPA ((RS)-α-amino-3-hydroxy-5-tert-butyl-4-isoxazolepropionic acid) was dissolved in distilled water and test compound was dissolved in a polyoxyl 40 hydrogenated castor oil (5% Cremophor RH™ 40 (BASF)).

Test compound was administered either i.v. 5, 30 or 120 min before or p.o. 30 min before an i.v. administration of 15 mg/kg of ATPA to 8 female NMRI mice per dose and the number of mice experiencing rigidity 5 min later was noted. An ED₅₀ value was calculated from at least three doses of test compound as the dose inhibiting 50% of the mice from having rigidity.

### Quisqualate-induced clonic seizures

Quisqualate given icv (intracerebroventricular) to DBA/2 mice induces clonic seizures which can be inhibited by both NMDA and non-NMDA receptor antagonists after i.v. administration.

### Method

Test compound was given i.v. 5 min before a 20 »g icv administration of quisqualate to 10 male DBA/2 mice (weighing 10-12 g) per dose. The number of mice experiencing clonic seizures within the next 2 min was noted. An ED₅₀ value was calculated as the dose inhibiting 50% of the mice from having clonic seizures.

### NMDA-induced clonic seizures

NMDA give icv to NMRI mice induces clonic seizures which can be inhibited by NMDA receptor antagonists.

### Method

Test compound was given i.v. 5 min before a 0.5 »g icv administration of NMDA to 10 male NMRI mice per dose. The number of mice experiencing clonic seizures within the next 2 min was noted. An ED₅₀ value was calculated as the dose inhibiting 50% of the mice from having clonic seizures.

### Cocaine-induced hypermotility

Quisqualate and kainate administered locally induce an increase in dopamine release in nucleus accumbens and nucleus caudatus accompanied by stereotype behaviour such as hyperlocomotion, rearing, sniffing and grooming. These effects can be inhibited by selective quisqualate antagonists administered locally by the micro-dialyses method.

Also the dopamine uptake inhibitor cocaine administered s.c. induce hypermotility which can be inhibited by an administration of the glutamate antagonist GDEE into nucleus accumbens.

For these reasons (and others) it has been concluded that non-NMDA receptors regulate the release of dopamine in nucleus accumbens and that non-NMDA receptor antagonists can alleviate the symptoms of psychosis.

### Method

Test compound was administered orally at doses of 0.1, 1, 10 and 30 mg/kg 30 min before the administration of 25 mg/kg cocaine i.p. to female NMRI mice and the locomotor activity of 2 mice per box was measured for the next 2 hours by use of 8 infrared photobeams per box. The mice had been adapted to the box for at least 16 hours to avoid exploratory motility (neophobia).

The quisqualate binding assay was performed as described by T. Honoré et al., Neuroscience Letters 54, 27-32 (1985).

The kainate binding assay was performed as described by T. Honoré et al., Neuroscience Letters 65, 47-52 (1986).

The glycine binding assay was performed as described by W. Frost White et al., Journal of Neurochemistry 53(2), 503-512 (1989).

### Pharmaceutical Compositions

The compounds of the invention, together with a conventional adjuvant, carrier, or diluent, may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, in the form of suppositories for rectal administration; or in the form of sterile injectable solutions for parenteral (including subcutaneous) use. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed. Tablets containing ten (10) milligrams of active ingredients or, more broadly, 0.1 to one hundred (100) milligrams, per tablet, are accordingly suitable representative unit dosage forms.

Solid forms of pharmaceutical compositions for p.o. administration and injectable solutions are preferred.

### Method of Treating

The compounds of this invention are extremely useful in the treatment of central nervous system disorders related to their biological activity. The compounds of this invention may accordingly be administered to a subject, including a human, in need of treatment, alleviation, or elimination of an indication associated with the biological activity of the compounds. This includes especially excitatory amino acid dependent psychosis, excitatory amino acid dependent anoxia, excitatory amino acid dependent ischemia, excitatory amino acid dependent convulsions and excitatory amino acid dependent migraine. Suitable dosage ranges are 0.1 to 1000 milligrams daily, 10-500 milligrams daily, and especially 30-100 milligrams daily, dependent as usual upon the exact mode of administration, Form in which administered, the indication toward which the administration is directed, the subject involved and the body weight of the subject involved, and further the preference and experience of the physician or veterinarian in charge.

### Chemical Examples

Some compounds of the invention are old, and others are novel chemical entities. In any-way the compounds of the invention may be prepared according to chemical methods which are well known.

### Example 1

### a) 1-phenyl-1H-indole-2,3-dione.

To a stirred solution of diphenylamine (3.2 g, 20 mmol) and 4-dimethylaminopyridine (10 mg) in chloroform (50 ml) was dropwise added oxalylchloride (3 ml). The resulting mixture was refluxed for 5 hours, whereafter it was cooled to room temperature and evaporated in vacuo.

The residue (oil) was redissolved in methylene chloride (50 ml) and dry AlCl₃ (3 g) was added. Stirring at room temperature was continued for 30 hours, whereafter ethanol (10 ml) followed by water (100 ml) were added. The organic phase was washed with saturated Na₂CO₃, dried over Na₂SO₄ and evaporated. The crystalline residue was stirred in ether (40 ml) and the product was filtered off. Yield: 2.65 g orange crystals, M.p. 139-141°C, litt. 138°C.

### b) The following 1H-indole-2,3-diones were prepared according to known literature procedures.

¹⁾Organic Synthesis Col Vol. I p. 327.
²⁾Martinet, J.: Compt. Rend. 166, 851, 1918.

4,6-ditrifluoromethyl-1H-indole-2,3-dione¹⁾, M.p. 162-165°C.
1H-benz[g]indole-2,3-dione²⁾, M.p. 242-245°C.
7-trifluoromethyl-1H-indole-2,3-dione¹⁾, M.p. 181-183°C.
1H-6,7,8,9-tetrahydro-benz[g]indole-2,3-dione, M.p. 224-226°C.
6-methoxy-1H-indole-2,3-dione, M.p. >310°C.
7-trifluoromethyl-1H-indole-2,3-dione, M.p. 180-184°C.

### c) 1-methyl-5-nitro-7-trifluoromethyl-1H-indole-2,3-dione.

To a stirred 10°C warm solution of KNO₃ (0.5 g) in 10 ml of conc. H₂SO₄ was dropwise added a solution of 1-methyl-7-trifluoromethyl-1H-indole-2,3-dione in 10 ml of conc. H₂SO₄. The addition was completed after 10 min, whereafter stirring was continued for 15 min at room temperature. The reaction mixture was poured on ice whereby the title compound precipitated as yellow crystals. The crystals were collected by filtration and washed with water. M.p. 168-169°C.

In a similar manner to c), the following nitro compounds were prepared:
5-nitro-1H-6,7,8,9-tetrahydro-benz[g]indole-2,3-dione, M.p. 232-236°C.
5-nitro-1-methyl-1H-benz[g]indole-2,3-dione, M.p. 255-258°C.

### d) 5,7-dinitro-1-methyl-1H-indole-2,3-dione.

To a stirred solution of 5,7-dinitro-1H-indole-2,3-dione (1.2 g) in dry dimethylformamide (20 ml) was added sodium hydride (0.24 g 55% in mineral oil). After the hydrogen evolution had ceased methyl iodide (0.37 ml) was added. Stirring at room temperature was continued for 2 hours, whereafter the crude product was precipitated as an oil by addition of water (100 ml) to the reaction mixture. The oil crystallized upon treatment with ether/pentane, M.p. 154-157°C.

In a similar manner to d), the following 1-alkyl- or 1-benzyl-1H-indole-2,3-diones were prepared.
5,7-dinitro-1-ethyl-1H-indole-2,3-dione, M.p. 135-140°C.
5-bromo-1-methyl-1H-indole-2,3-dione, M.p. 157-160°C.
1H-1-methyl-6,7,8,9-tetrahydro-benz[g]indole-2,3-dione, M.p. 157-160°C.
5,7-dibromo-1-methyl-1H-indole-2,3-dione, M.p. 170-173°C.
5,6-dichloro-1-methyl-1H-indole-2,3-dione, M.p. 180-184°C.
4,5-dichloro-1-methyl-1H-indole-2,3-dione, M.p. 237-239°C.
1-methyl-5-nitro-1H-indole-2,3-dione, M.p. 196-199°C.
1-benzyl-5,7-dinitro-1H-indole-2,3-dione, M.p. 127-131°C.
4,6-ditrifluoromethyl-1-methyl-1H-indole, M.p. 93-94°C.
1-methyl-7-trifluoromethyl-1H-indole-2,3-dione, M.p. 120-122°C.
6-methoxy-1-methyl-1H-indole-2,3-dione, M.p. 175-178°C.
5,7-dinitro-1-(ethoxycarbonylmethyl)-1H-indole-2,3-dione, (oil).
1-methyl-1H-benz[g]indole-2,3-dione, M.p. 122-126°C.
1-(ethoxycarbonylmethyl)-1H-indole-2,3-dione, M.p. 115-119°C.
5,7-dibromo-1-(ethoxycarbonylmethyl)-1H-indole-2,3-dione, M.p. 97-102°C.
1-methyl-1H-6,7,8,9-tetrahydrobenz[g]indole-2,3-dione, M.p. 160-165°C.

### Example 2

5,7-dinitro-1-methyl-1H-indole-2,3-dione-3-(O-methyloxime).

5,7-dinitro-1-methyl-1H-indole-2,3-dione (0.4 g), O-methyl-hydroxylamine, hydrochloride (0.16 g) and sodium carbonate (0.2 g) was stirred at room temperature in ethanol (5 ml) for one hour, whereafter acetic acid (0.5 ml) followed by water (50 ml) were added. The mixture was cooled on ice and the crystalline product was obtained by filtration, M.p. 145-151°C.

The following O-alkyloximes were prepared in a similar manner starting from the appropriate indole-2,3-diones.
5,7-dinitro-1H-indole-2,3-dione-3-(O-methyloxime), M.p. 236-239°C.
5,7-dinitro-1-ethyl-1H-indole-2,3-dione-3-(O-methyloxime), M.p. 156-159°C.
5-nitro-3-(O-methyloxime)-1H-indole-2,3-dione, M.p. 293-295°C.
1-phenyl-1H-indole-2,3-dione-3-(O-methyloxime), M.p. 151-153°C.
1H-indole-2,3-dione-3-(O-methyloxime), M.p. 168-171°C.
5,7-dibromo-1-methyl-1H-indole-2,3-dione-3-(O-methyloxime), M.p. 170-172°C.
5,7-dibromo-1H-indole-2,3-dione-3-(O-methyloxime), M.p. 279°C (decomp.).
1-methyl-5-nitro-1H-indole-2,3-dione-3-(O-methyloxime), M.p. 167-170°C.
5,6-dichloro-1-methyl-1H-indole-2,3-dione-3-(O-methyloxime), M.p. 202-204°C.
4,5-dichloro-1-methyl-1H-indole-2,3-dione-3-(O-methyloxime), M.p. 180-183°C.
5,7-dinitro-1-benzyl-1H-indole-2,3-dione-3-(O-methyloxime), M.p. 181-185°C.
4,5-ditrifluoromethyl-1-methyl-1H-indole-2,3-dione-3-(O-methyloxime), M.p. 99-100°C.
5-nitro-7-trifluoromethyl-1-methyl-1H-indole-2,3-dione-3-(O-methyloxime), M.p. 160-161°C.
5-nitro-7-trifluoromethyl-1H-indole-2,3-dione-3-(O-methyloxime), M.p. 225-228°C.
5,7-dinitro-6-methoxy-1-methyl-1H-indole-2,3-dione-3-(O-methyloxime), M.p. 145-148°C.
5,7-dinitro-1-(O-ethylcarboxymethyl)-1H-indole-2,3-dione-3-(O-methyloxime), M.p. 115-117°C.
5-nitro-1-methyl-1H-benz[g]indole-2,3-dione-3-(O-methyloxime), M.p. 255-258°C.
5-bromo-1-(ethoxycarbonylmethyl)-1H-indole-2,3-dione-3-(O-methyloxime), M.p. 201-204°C.
5,7-dibromo-1-(ethoxycarbonylmethyl)-1H-indole-2,3-dione-3-(O-methyloxime), M.p. 137-138°C.
5-methyl-1-(methoxycarbonylmethyl)-1H-indole-2,3-dione-3-(O-methyloxime), M.p. 127-131°C.
5-nitro-1-methyl-1H-6,7,8,9-tetrahydro-benz[g]indole-2,3-dione-3-(O-methyloxime), M.p. 220-224°C.

Substitution of O-methyl-hydroxylamine hydrochloride in the process by hydroxylamine hydrochloride, afforded the following oximes.
1H-benz[g]indole-2,3-dione-3-oxime, M.p. 248-250°C.
5,7-dinitro-1-methyl-1H-indole-2,3-dione-3-oxime, M.p. 244°C (decomp.).
5,7-dibromo-1H-indole-2,3-dione-3-oxime, M.p. 240-242°C.
5-bromo-1-methyl-1H-indole-2,3-dione-3-oxime, M.p. 213-215°C.
5,7-dinitro-1H-indole-2,3-dione-3-oxime, M.p. 240-242°C.
5-bromo-7-nitro-1H-indole-2,3-dione-3-oxime, M.p. 254-256°C.
5-bromo-1H-indole-2,3-dione-3-oxime, M.p. 250-251°C.
5-nitro-1H-indole-2,3-dione-3-oxime, M.p. 243-245°C.
5-methyl-1H-indole-2,3-dione-3-oxime, M.p. 203-206°C.
1H-indole-2,3-dione-3-oxime, M.p. 234-236°C.
1-methyl-6,7,8,9-tetrahydro-1H-benz[g]indole-2,3-dione-3-oxime, M.p. 230-232°C.
5,6-dichloro-1-methyl-1H-indole-2,3-dione-3-oxime, M.p. 232-236°C.
4-phenyl-7-methoxy-1H-indole-2,3-dione-3-oxime M.p. 201-204°C.
4,5-dichloro-1H-indole-2,3-dione-3-oxime, M.p. 245-247°C.
1-phenyl-1H-indole-2,3-dione-3-oxime, M.p. 166-170°C.
4,5-dichloro-1-methyl-1H-indole-2,3-dione-3-oxime, M.p. 140-142°C.
5-nitro-1H-benz[g]indole-2,3-dione-3-oxime, M.p. 197-199°C.
5-nitro-7-trifluoromethyl-1-methyl-1H-indole-2,3-dione-3-oxime, M.p. 204-205°C.
5-nitro-7-trifluoromethyl-1H-indole-2,3-dione-3-oxime, M.p. 230-232°C.
5-nitro-1H-6,7,8,9-tetrahydro-benz[g]indole-2,3-dione-3-oxime, M.p. 205-210°C.
5-fluoro-7-nitro-1H-indole-2,3-dione-3-oxime, M.p. 260-262°C.
5,7-dinitro-1-(ethoxycarbonylmethyl)-1H-indole-2,3-dione-3-oxime, M.p. 217-220°C.
1-(ethoxycarbonylmethyl)-1H-indole-2,3-dione-3-oxime, M.p. 183-185°C.
5-bromo-1-(ethoxycarbonylmethyl)-1H-indole-2,3-dione-3-oxime, M.p. 178-181°C.
5,7-dinitro-1H-indole-2,3-indole-2,3-dione-3-oxime, M.p. 195-197°C.

Substitution ox hydroxylamine hydrochloride in the process by O-benzylhydroxylamine, afforded the following compounds.
5,7-dinitro-1H-indole-2,3-dione-3-(O-benzyloxime), M.p. 197-199°C.
5,7-dinitro-1H-1-benzyl-indole-2,3-dione-3-(O-benzyloxime), M.p. 148-150°C.
5,7-dinitro-1-methyl-1H-indole-2,3-dione-3-(O-benzyloxime), M.p. 120-125°C.
5,7-dinitro-1-(ethoxycarbonylmethyl)-1H-indole-2,3-dione-3-(O-benzyloxime), M.p. 100-102°C.
5-nitro-1-(ethoxycarbonylmethyl)-1H-indole-2,3-dione-3-(O-benzyloxime), M.p. 185-187°C.

The following compounds were prepared according to literature procedures:
1-methoxy-1H-indole-2,3-dione-3-oxime, M.p. 166-168°C.¹⁾
1-acetyl-5-bromo-1H-indole-2,3-dione, M.p. 133-135°C.
1-hydroxy-1H-indole-2,3-dione-3-oxime, M.p. 217-221°C.¹⁾
¹⁾A. Reiszert, Ber. vol. 41, 3921.

### Example 3

### a) 1-carboxymethyl-1H-indole-2,3-dione.

8.39 g (36 mmol) 1-(ethoxycarbonylmethyl)-1H-indole-2,3-dione and 4N NaOH (10 ml, 40 mmol) were dissolved in 30 ml H₂O and 10 ml absolute ethanol and the mixture was heated at reflux for 30 minutes. The reaction mixture was cooled and added excess hydrochloric acid. The precipitate was isolated. Yield of title compound is 6.2 g.

### b) 1-chlorocarbonylmethyl-1H-indole-2,3-dione.

1 g of the product prepared under a) was suspended in toluene (10 ml) and SOCl₂ (1.0 ml, 13.78 mmol) was added. The mixture was stirred at RT for 2 hours and additionally at 70°C for 30 minutes and thereafter at reflux for 1 hour. The reaction mixture was stirred at RT overnight whereafter the precipitated yellow crystals were isolated and washed with toluene. Yield of title compound was 1.3 g including solvent content.

### c) 1-aminocarbonylmethyl-1H-indole-2,3-dione.

The product prepared under b) was dissolved in dry THF (50 ml) and to the solution was added liquid NH₃. The resulting mixture was stirred overnight at RT. The precipitated orange crystals were isolated and were washed with water. Yield or title compound was 0.46 g.

### d) 1-cyanomethyl-1H-indole-2,3-dione.

Triphenylphosphine (0.75 g, 2.84 mmol) was dissolved in methylenechloride and to the solution was added dropwise to Br₂ (0.15 ml, 2.84 mmol) in methyleneohloride (20 ml). To this mixture the product prepared under c) was added, and thereafter triethylamine (0.8 ml, 5.63 mmol) was added dropwise. The mixture was stirred for 30 minutes. The reaction mixture was evaporated in vacuo and the residue was taken up in ether. The precipitate from this mixture was filtered off and the ether solution was washed with water and dried (MgSO₄). The ether solution was evaporated in vacuo and the residue was washed with isopropanol. Yield of title compound was 0.11 g. M.p. 125-128°C.

### e) 1-(acetamideoxime-2-yl)-1H-indole-2,3-dione-3-oxime.

The product prepared under d) (90 mg, 0.48 mmol), hydroxylamine hydrochloride (70 mg, 1.06 mmol), potassium carbonate (150 mg, 1.06 mmol) and methanol (10 ml) were mixed and the mixture was stirred at RT overnight. The reaction mixture was evaporated in vacuo. The residue was washed with water containing small amounts of acetic acid. Yield of title compound was 70 mg. M.p. 227-229°C.

It is thus seen that the present invention provides a new and convenient process for the production of indole-2,3-dione-3-oxime compounds, certain novel indole-2,3-dione-3-oxime compounds which are useful as excitatory amino acid antagonists, pharmaceutical-compositions useful as excitatory amino acid antagonists comprising certain indole-2,3-dione-3-oxime compounds, and a method of antagonizing the biological effects of excitatory amino acids in a subject in need thereof comprising the step of administering certain indole-2,3-dione-3-oxime compounds or a pharmaceutical composition comprising the same together with a pharmaceutically-acceptable diluent or carrier, all having the foregoing characteristics and advantages.

## Claims (Claims for the following Contracting State(s): AT, BE, CH, LI, DE, DK, FR, GB, IT, LU, NL, SE)

1. A compound having the formula 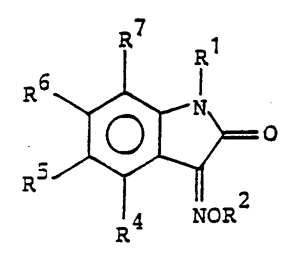 wherein R¹ is hydrogen, C₁₋₆-alkyl which may be branched, C₃₋₇-cyclo alkyl, benzyl, acyl, hydroxy, C₁₋₆-alkoxy, CH₂CO₂R' wherein R' is hydrogen or C₁₋₆-alkyl which may be branched, CH₂CN, CH₂CONR^{IV}R^{V} wherein R^{IV} and R^{V} independently are hydrogen or C₁₋₆-alkyl, or CH₂C(=NOH)NH₂;
R² is hydrogen, benzyl, C₁₋₆-alkyl which may be branched, or C₃₋₇-cycloalkyl;
R⁵ is NO₂, F, CF₃, SO₂NR''R''' wherein R'' and R''' independently are hydrogen or C₁₋₆-alkyl, or CN; and R⁴, R⁶, R⁷ independently are hydrogen, C₁₋₆-alkyl which may be branched, phenyl, halogen, C₁₋₆-alkoxy, NO₂, CN, SO₂NR''R''' wherein R'' and R''' independently are hydrogen or C₁₋₆-alkyl, or CF₃, or R⁶ and R⁷ together form an additional 4 to 7 membered ring which may be aromatic or partial saturated and which may be substituted with halogen, NO₂, CF₃, CN, SO₂NR''R''' wherein R'' and R''' independently are hydrogen or C₁₋₆-alkyl, and R⁴ has the meaning set forth above, with the proviso that R⁵ is different from NO₂ and F when R¹, R⁴, R⁶ and R⁷ are hydrogen and R² is hydrogen or benzyl; and that R⁵ is different from NO₂ when R¹, R², R⁴ and R⁶ are hydrogen and R⁷ is NO₂.

2. The compound according to claim 1 wherein R⁵ is F, NO₂, CN, CF₃, or SO₂NR''R''' wherein R'' and R''' independently are hydrogen or C₁₋₆-alkyl, and wherein R⁶ and R⁷ together form an additional 4 to 7 membered ring which may be aromatic or partial saturated and which may be substituted with halogen, NO₂, CF₃, CN, SO₂NR''R''' wherein R'' and R''' independently are hydrogen or C₁₋₆-alkyl.

3. The compound according to claim 2 wherein the additional ring formed by R⁶ and R⁷ is substituted with halogen, NO₂, CF₃, CN or SO₂NR''R''' wherein R'' and R''' independently are hydrogen or C₁₋₆-alkyl.

4. The compound according to Claim 1, which is 5-nitro-1H-benz[ g] -indole-2,3-dione-3-oxime.

5. The compound according to Claim 1, which is 5,7-dinitro-1-methyl-1H-indole-2,3-dione-3-(0-methyloxime).

6. The compound according to Claim 1, which is 5-nitro-1H-6,7,8,9-tetrahydro-benz[ g] indole-2,3-dione-3-oxime.

7. A pharmaceutical composition comprising an effective excitatory amino acid antagonizing amount of a compound having the formula 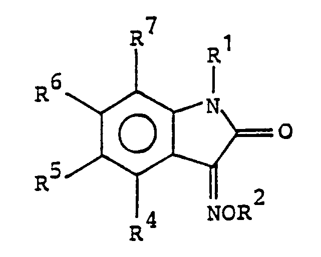 wherein R¹ is hydrogen, C₁₋₆-alkyl which may be branched, C₃₋₇-cyclo alkyl, benzyl, acyl, hydroxy, C₁₋₆-alkoxy, CH₂CO₂R' wherein R' is hydrogen or C₁₋₆-alkyl which may be branched, CH₂CN, CH₂CONR^{IV}R^{V} wherein R^{IV} and R^{V} independently are hydrogen or C₁₋₆-alkyl, or CH₂C(=NOH)NH₂;
R² is hydrogen, benzyl, C₁₋₆-alkyl which may be branched, or C₃₋₇-cycloalkyl;
R⁵ is NO₂, F, CF₃, SO₂NR''R''' wherein R'' and R''' independently are hydrogen or C₁₋₆-alkyl, or CN; and R⁴, R⁶, R⁷ independently are hydrogen, C₁₋₆-alkyl which may be branched, phenyl, halogen, C₁₋₆-alkoxy, NO₂, CN, SO₂NR''R''' wherein R'' and R''' independently are hydrogen or C₁₋₆-alkyl, or CF₃, or R⁶ and R⁷ together form an additional 4 to 7 membered ring which may be aromatic or partial saturated and which may be substituted with halogen, NO₂, CF₃, CN, SO₂NR''R''' wherein R'' and R''' independently are hydrogen or C₁₋₆-alkyl, and R⁴ has the meaning set forth above.

8. A method of preparing the compound according to Claim 1, comprising the step of reacting a compound of the formula 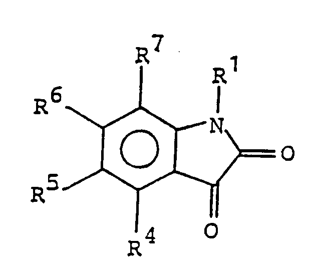 wherein R¹, R⁴, R⁵, R⁶, and R⁷ have the meaning set forth in Claim 1, with a compound having the formula NH₂OR², wherein R² has the meaning set forth in Claim 1.

9. The use of an indole-2,3-dione-3-oxime compound having the formula 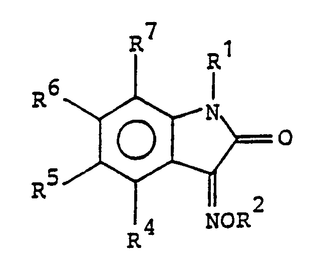 wherein
R¹ is hydrogen, C₁₋₆-alkyl which may be branched, C₃₋₇-cycloalkyl, benzyl, phenyl
acyl, hydroxy, C₁₋₆-alkoxy, CH₂CO₂R' wherein R' is hydrogen or C₁₋₆-alkyl which may be branched, CH₂CN, CH₂CONR^{IV}R^{V} wherein R^{IV} and R^{V} independently are hydrogen or C₁₋₆-alkyl, or CH₂C(=NOH)NH₂;
R² is hydrogen, benzyl, C₁₋₆-alkyl which may be branched, or C₃₋₇-cycloalkyl;
R⁵ is NO₂, F, CF₃, SO₂NR''R''' wherein R'' and R''' independently are hydrogen or C₁₋₆-alkyl, or CN; and R⁴, R⁶, R⁷ independently are hydrogen, C₁₋₆-alkyl which may be branched, phenyl, halogen, C₁₋₆-alkoxy, NO₂, CN, SO₂NR''R''' wherein R'' and R''' independently are hydrogen or C₁₋₆-alkyl, or CF₃, or R⁶ and R⁷ together form an additional 4 to 7 membered ring which may be aromatic or partial saturated and which may be substituted with halogen, NO₂, CF₃, CN, SO₂NR''R''' wherein R'' and R''' independently are hydrogen or C₁₋₆-alkyl, and R⁴ has the meaning set forth above, for the preparation of a medicament useful in the treatment of conditions sensitive to an excitatory amino acid.

10. The use according to Claim 9 wherein at least one of R⁴, R⁵, R⁶ and R⁷ is NO₂, CF₃, CN, SO₂NR''R''', or halogen and R¹, R², R⁴, R⁵, R⁶, R⁷, R'', and R''' otherwise have the meanings set forth in claim 1.

11. The use according to Claim 9 wherein R⁵ is NO₂, F, CF₃, or CN.

## Claims (Claims for the following Contracting State(s): ES)

1. A method for preparing a compound having the formula 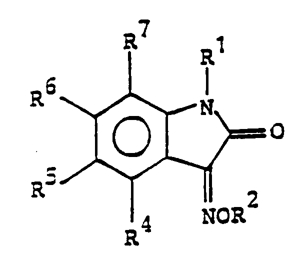 wherein R¹ is hydrogen, C₁₋₆-alkyl which may be branched, C₃₋₇-cyclo alkyl, benzyl, acyl,
hydroxy, C₁₋₆-alkoxy, CH₂CO₂R' wherein R' is hydrogen or C₁₋₆-alkyl which may be branched, CH₂CN, CH₂CONR^{IV}R^{V} wherein R^{IV} and R^{V} independently are hydrogen or C₁₋₆-alkyl, or CH₂C(=NOH)NH₂;
R² is hydrogen, benzyl, C₁₋₆-alkyl which may be branched, or C₃₋₇-cycloalkyl;
R⁵ is NO₂, F, CF₃, SO₂NR''R''' wherein R'' and R''' independently are hydrogen or C₁₋₆-alkyl or CN; and R⁴, R⁶, R⁷ independently are hydrogen, C₁₋₆-alkyl, which may be branched, phenyl, halogen, C₁₋₆-alkoxy, NO₂, CN, SO₂NR''R''' wherein R'' and R''' independently are hydrogen or C₁₋₆-alkyl, or CF₃, or R⁶ and R⁷ together form an additional 4 to 7 membered ring which may be aromatic or partial saturated and which may be substituted with halogen, NO₂, CF₃ CN, SO₂NR''R''' wherein R'' and R''' independently are hydrogen or C₁₋₆-alkyl, and R⁴ has the meaning set forth above, with the proviso that R⁵ is different from NO₂ and F when R¹, R⁴, R⁶ and R⁷ are hydrogen and R² is hydrogen or benzyl; and that R⁵ is different from NO₂ when R¹, R², R⁴ and R⁶ are hydrogen and R⁷ is NO₂, comprising the step of reacting a compound of the formula 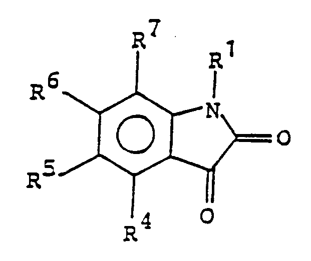 wherein R¹, R⁴, R⁵, R⁶, and R⁷ have the meaning set forth above, with a compound having the formula NH₂OR², wherein R² has the meaning as set forth above.

2. The process according to claim 1, wherein R⁵ is F, NO₂, CN, CF₃, or SO₂NR''R''' wherein R'' and R''' independently are hydrogen or C₁₋₆-alkyl, and wherein R⁶ and R⁷ together form an additional 4 to 7 membered ring which may be aromatic or partial saturated and which may be substituted with halogen, NO₂, CF₃, CN, SO₂NR''R''' wherein R'' and R''' independently are hydrogen or C₁₋₆-alkyl.

3. The process according to claim 2, wherein the additional ring formed by R⁶ and R⁷ is substituted with halogen, NO₂, CF₃, CN or SO₂NR''R''' wherein R'' and R''' independently are hydrogen or C₁₋₆-alkyl.

4. The process according to claim 1, wherein the prepared compound is 5-nitro-1H-benz[ g] -indole-2,3-dione-3-oxime.

5. The process according to claim 1, wherein the prepared compound is 5,7-dinitro-1-methyl-1H-indole-2,3-dione-3-(0-methyloxime).

6. The process according to claim 1, wherein the prepared compound is 5-nitro-1H-6,7,8,9-tetrahydro-benz[ g] indole-2,3-dione-3-oxime.

7. A process for preparing a pharmaceutical composition comprising an effective excitatory amino acid antagonizing amount of a compound having the formula 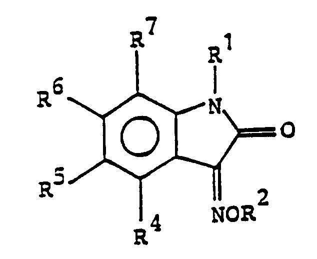 wherein R¹ is hydrogen, C₁₋₆-alkyl which may be branched, C₃₋₇-cyclo alkyl, benzyl, acyl,
hydroxy, C₁₋₆-alkoxy, CH₂CO₂R' wherein R' is hydrogen or C₁₋₆-alkyl which may be branched, CH₂CN, CH₂CONR^{IV}R^{V} wherein R^{IV} and R^{V} independently are hydrogen or C₁₋₆-alkyl, or CH₂C(=NOH)NH₂;
R² is hydrogen, benzyl, C₁₋₆-alkyl which may be branched, or C₃₋₇-cycloalkyl;
R⁵ is NO₂, F, CF₃, SO₂NR''R''' wherein R'' and R''' independently are hydrogen or C₁₋₆-alkyl or CN; and R⁴, R⁶ R⁷ independently are hydrogen, C₁₋₆-alkyl which may be branched, phenyl, halogen, C₁₋₆-alkoxy, NO₂, CN, SO₂NR''R''' wherein R'' and R''' independently are hydrogen or C₁₋₆-alkyl, or CF₃, or R⁶ and R⁷ together form an additional 4 to 7 membered ring which may be aromatic or partial saturated and which may be substituted with halogen, NO₂, CF₃, CN, SO₂NR''R''' wherein R'' and R''' independently are hydrogen or C₁₋₆-alkyl, and R⁴ has the meaning set forth above, by combining the compound with conventional additives in a manner known per se.

## Claims (Claims for the following Contracting State(s): GR)

1. A compound having the formula 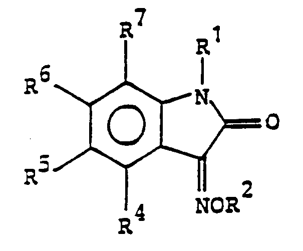 wherein R¹ is hydrogen, C₁₋₆-alkyl which may be branched, C₃₋₇-cyclo alkyl, benzyl, acyl,
hydroxy, C₁₋₆-alkoxy, CH₂CO₂R' wherein R' is hydrogen or C₁₋₆-alkyl which may be branched, CH₂CN, CH₂CONR^{IV}R^{V} wherein R^{IV} and R^{V} independently are hydrogen or C₁₋₆-alkyl, or CH₂C(=NOH)NH₂;
R² is hydrogen, benzyl, C₁₋₆-alkyl which may be branched, or C₃₋₇-cycloalkyl;
R⁵ is NO₂, F, CF₃, SO₂NR''R''' wherein R'' and R''' independently are hydrogen or C₁₋₆-alkyl or CN; and R⁴, R⁶, R⁷ independently are hydrogen, C₁₋₆-alkyl, which may be branched, phenyl, halogen, C₁₋₆-alkoxy, NO₂, CN, SO₂NR''R''' wherein R'' and R''' independently are hydrogen or C₁₋₆-alkyl, or CF₃, or R⁶ and R⁷ together form an additional 4 to 7 membered ring which may be aromatic or partial saturated and which may be substituted with halogen, NO₂, CF₃ CN, SO₂NR''R''' wherein R'' and R''' independently are hydrogen or C₁₋₆-alkyl, and R⁴ has the meaning set forth above, with the proviso that R⁵ is different from NO₂ and F when R¹, R⁴, R⁶ and R⁷ are hydrogen and R² is hydrogen or benzyl; and that R⁵ is different from NO₂ when R¹, R², R⁴ and R⁶ are hydrogen and R⁷ is NO₂.

2. The compound according to claim 1, wherein R⁵ is F, NO₂, CN, CF₃, or SO₂NR''R''' wherein R'' and R''' independently are hydrogen or C₁₋₆-alkyl, and wherein R⁶ and R⁷ together form an additional 4 to 7 membered ring which may be aromatic or partial saturated and which may be substituted with halogen, NO₂, CF₃, CN, SO₂NR''R''' wherein R'' and R''' independently are hydrogen or C₁₋₆-alkyl.

3. The compound according to claim 2, wherein the additional ring formed by R⁶ and R⁷ is substituted with halogen, NO₂, CF₃, CN or SO₂NR''R''' wherein R'' and R''' independently are hydrogen or C₁₋₆-alkyl.

4. The compound according to claim 1, which is 5-nitro-1H-benz[ g] -indole-2,3-dione-3-oxime.

5. The compound according to claim 1, which is 5,7-dinitro-1-methyl-1H-indole-2,3-dione-3-(0-methyloxime).

6. The compound according to claim 1, which is 5-nitro-1H-6,7,8,9-tetrahydro-benz[ g] indole-2,3-dione-3-oxime.

7. A process for preparing a pharmaceutical composition comprising an effective excitatory amino acid antagonizing amount of a compound having the formula 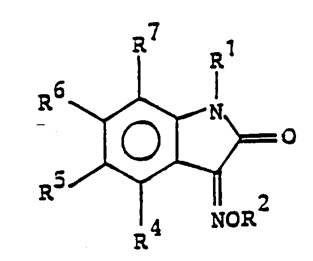 wherein R¹ is hydrogen, C₁₋₆-alkyl which may be branched, C₃₋₇-cyclo alkyl, benzyl, acyl,
hydroxy, C₁₋₆-alkoxy, CH₂CO₂R' wherein R' is hydrogen or C₁₋₆-alkyl which may be branched, CH₂CN, CH₂CONR^{IV}R^{V} wherein R^{IV} and R^{V} independently are hydrogen or C₁₋₆-alkyl, or CH₂C(=NOH)NH₂;
R² is hydrogen, benzyl, C₁₋₆-alkyl which may be branched, or C₃₋₇-cycloalkyl;
R⁵ is NO₂, F, CF₃, SO₂NR''R''' wherein R'' and R''' independently are hydrogen or C₁₋₆-alkyl or CN; and R⁴, R⁶, R⁷ independently are hydrogen, C₁₋₆-alkyl which may be branched, phenyl, halogen, C₁₋₆-alkoxy, NO₂, CN, SO₂NR''R''' wherein R'' and R''' independently are hydrogen or C₁₋₆-alkyl, or CF₃, or R⁶ and R⁷ together form an additional 4 to 7 membered ring which may be aromatic or partial saturated and which may be substituted with halogen, NO₂, CF₃, CN, SO₂NR''R''' wherein R'' and R''' independently are hydrogen or C₁₋₆-alkyl, and R⁴ has the meaning set forth above by combining the compound with conventional additives in a manner known per se.

8. A method of preparing the compound according to claim 1, comprising the step of reacting a compound of the formula 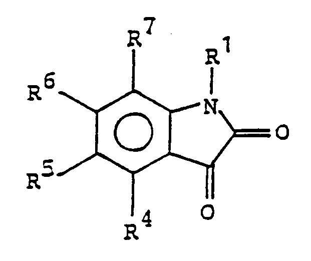 wherein R¹, R⁴, R⁵, R⁶ and R⁷ have the meaning set forth in claim 1, with a compound having the formula NH₂OR², wherein R² has the meaning set forth in claim 1.

9. The use of an indole-2,3-dione-3-oxime compound having the formula 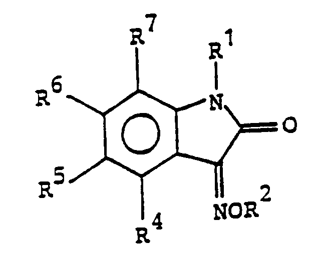 wherein R¹ is hydrogen, C₁₋₆-alkyl which may be branched, C₃₋₇-cycloalkyl, benzyl, phenyl, acyl,
hydroxy, C₁₋₆-alkoxy, CH₂CO₂R' wherein R' is hydrogen or C₁₋₆-alkyl which may be branched, CH₂CN, CH₂CONR^{IV}R^{V} wherein R^{IV} and R^{V} independently are hydrogen or C₁₋₆-alkyl, or CH₂C(=NOH)NH₂;
R² is hydrogen, benzyl, C₁₋₆-alkyl which may be branched, or C₃₋₇-cycloalkyl;
R⁵ is NO₂, F, CF₃, SO₂NR''R''' wherein R'' and R''' independently are hydrogen or C₁₋₆-alkyl or CN; and R⁴, R⁶, R⁷ independently are hydrogen, C₁₋₆-alkyl which may be branched, phenyl, halogen, C₁₋₆-alkoxy, NO₂, CN, SO₂NR''R''' wherein R'' and R''' independently are hydrogen or C₁₋₆-alkyl, or CF₃, or R⁶ and R⁷ together form an additional 4 to 7 membered ring which may be aromatic or partial saturated and which may be substituted with halogen, NO₂, CF₃, CN, SO₂NR''R''' wherein R'' and R''' independently are hydrogen or C₁₋₆-alkyl, and R⁴ has the meaning set forth above, for the preparation of a medicament useful in the treatment of conditions sensitive to an excitatory amino acid.

10. The use according to claim 9 wherein at least one of R⁴, R⁵, R⁶ and R⁷ is NO₂, CF₃, CN, SO₂NR''R''', or halogen and R¹, R², R⁴, R⁵, R⁶, R⁷, R'', and R''' otherwise have the meanings set forth in claim 1.

11. The use according to claim 9 wherein R⁵ is NO₂, F, CF₃, or CN.

## Patentansprüche (Patentansprüche für folgende(n) Vertragsstaat(en): AT, BE, CH, LI, DE, DK, FR, GB, IT, LU, NL, SE)

1. Verbindung mit der Formel 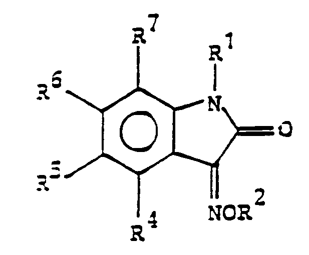 worin R¹ Wasserstoff, C₁₋₆-Alkyl, welches verzweigt sein kann, C₃₋₇-Cycloalkyl, Benzyl, Acyl, Hydroxy, C₁₋₆-Alkoxy, CH₂CO₂R', worin R' Wasserstoff oder C₁₋₆-Alkyl, welches verzweigt sein kann, ist, CH₂CN, CH₂CONR^{IV}R^{V}, worin R^{IV} und R^{V} unabhängig voneinander Wasserstoff oder C₁₋₆-Alkyl sind, oder CH₂C(=NOH)NH₂ ist;
R² Wasserstoff, Benzyl, C₁₋₆-Alkyl, welches verzweigt sein kann, oder C₃₋₇-Cycloalkyl ist;
R⁵ NO₂, F, CF₃,SO₂NR''R''', worin R'' und R''' unabhängig voneinander Wasserstoff oder C₁₋₆-Alkyl sind, oder CN ist; und
R⁴, R⁶, R⁷ unabhängig voneinander Wasserstoff, C₁₋₆-Alkyl, welches verzweigt sein kann, Phenyl, Halogen, C₁₋₆-Alkoxy, NO₂, CN, SO₂NR''R''', worin R'' und R''' unabhängig voneinander Wasserstoff oder C₁₋₆-Alkyl sind, oder CF₃ sind, oder R⁶ und R⁷ zusammen einen zusätzlichen 4- bis 7-gliedrigen Ring bilden, welcher aromatisch oder teilweise gesättigt sein kann und welcher mit Halogen, NO₂, CF₃, CN, SO₂NR''R''', worin R'' und R''' unabhängig voneinander Wasserstoff oder C₁₋₆-Alkyl sind, substituiert sein kann, und R⁴ die vorstehend angegebene Bedeutung hat, mit der Maßgabe, daß R⁵ von NO₂ und F verschieden ist, wenn R¹, R⁴, R⁶ und R⁷ Wasserstoff sind, und R² Wasserstoff oder Benzyl ist; und daß R⁵ von NO₂ verschieden ist, wenn R¹, R², R⁴ und R⁶ Wasserstoff sind und R⁷ NO₂ ist.

2. Verbindung nach Anspruch 1, worin R⁵ F, NO₂, CN, CF₃ oder SO₂NR''R''', worin R'' und R''' unabhängig voneinander Wasserstoff oder C₁₋₆-Alkyl sind, ist, und worin R⁶ und R⁷ zusammen einen zusätzlichen 4- bis 7-gliedrigen Ring bilden, welcher aromatisch oder teilweise gesättigt sein kann und welcher mit Halogen, NO₂, CF₃, CN, SO₂NR''R''', worin R'' und R''' unabhängig voneinander Wasserstoff oder C₁₋₆-Alkyl sind, substituiert sein kann.

3. Verbindung nach Anspruch 2, worin der zusätzliche Ring, der durch R⁶ und R⁷ gebildet wird, mit Halogen, NO₂, CF₃, CN, oder SO₂NR''R''' substituiert ist, worin R'' und R''' unabhängig voneinander Wasserstoff oder C₁₋₆-Alkyl sind.

4. Verbindung nach Anspruch 1, welche 5-Nitro-1H-benz[g]indol-2,3-dion-3-oxim ist.

5. Verbindung nach Anspruch 1, welche 5,7-Dinitro-1-methyl-1H-indol-2,3-dion-3-(O-methyloxim) ist.

6. Verbindung nach Anspruch 1, welche 5-Nitro-1H-6,7,8,9-tetrahydro-benz[g]indol-2,3-dion-3-oxim ist.

7. Pharmazeutische Zusammensetzung, umfassend eine wirksame excitatorischen Aminosäuren entgegenwirkende Menge einer Verbindung mit der Formel: 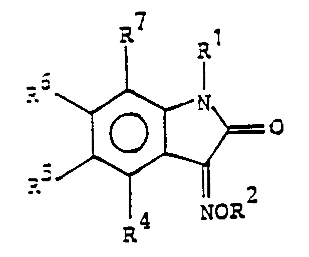 worin R¹ Wasserstoff, C₁₋₆-Alkyl, welches verzweigt sein kann, C₃₋₇-Cycloalkyl, Benzyl, Acyl, Hydroxy, C₁₋₆-Alkoxy, CH₂CO₂R', worin R' Wasserstoff oder C₁₋₆-Alkyl, welches verzweigt sein kann, ist, CH₂CN, CH₂CONR^{IV}R^{V}, worin R^{IV} und R^{V} unabhängig voneinander Wasserstoff oder C₁₋₆-Alkyl sind, oder CH₂C(=NOH)NH₂ ist;
R² Wasserstoff, Benzyl, C₁₋₆-Alkyl, welches verzweigt sein kann, oder C₃₋₇-Cycloalkyl ist;
R⁵ NO₂, F, CF₃, SO₂NR''R''', worin R'' und R''' unabhängig voneinander Wasserstoff oder C₁₋₆-Alkyl sind, oder CN ist; und
R⁴, R⁶, R⁷ unabhängig voneinander Wasserstoff, C₁₋₆-Alkyl, welches verzweigt sein kann, Phenyl, Halogen, C₁₋₆-Alkoxy, NO₂, CN, SO₂NR''R''', worin R'' und R''' unabhängig voneinander Wasserstoff oder C₁₋₆-Alkyl sind, oder CF₃ sind, oder R⁵ und R⁷ zusammen einen zusätzlichen 4- bis 7-gliedrigen Ring bilden, welcher aromatisch oder teilweise gesättigt sein kann und welcher mit Halogen, NO₂, CF₃, CN, SO₂NR''R''', worin R'' und R''' unabhängig voneinander Wasserstoff oder C₁₋₆-Alkyl sind, substituiert sein kann, und R⁴ die vorstehend angegebene Bedeutung hat.

8. Verfahren zum Herstellen der Verbindung nach Anspruch 1, umfassend den Schritt des Umsetzens einer Verbindung der Formel 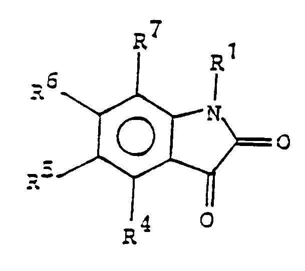 worin R¹, R⁴, R⁵, R⁶ und R⁷ die in Anspruch 1 angegebene Bedeutung haben, mit einer Verbindung mit der Formel NH₂OR², worin R² die in Anspruch 1 angegebene Bedeutung hat.

9. Verwendung einer Indol-2,3-dion-3-oxim-Verbindung mit der Formel 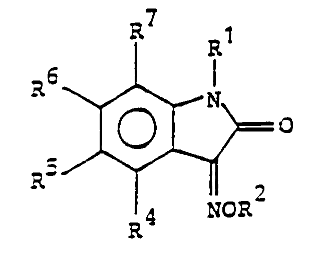 worin R¹ Wasserstoff, C₁₋₆-Alkyl, welches verzweigt sein kann, C₃₋₇-Cycloalkyl, Benzyl, Phenyl, Acyl, Hydroxy, C₁₋ ₆-Alkoxy, CH₂CO₂R', worin R' Wasserstoff oder C₁₋₆-Alkyl, welches verzweigt sein kann, ist, CH₂CN, CH₂CONR^{IV}R^{V}, worin R^{IV} und R^{V} unabhängig voneinander Wasserstoff oder C₁₋₆-Alkyl sind, oder CH₂C(=NOH)NH₂ ist;
R² Wasserstoff, Benzyl, C₁₋₆-Alkyl, welches verzweigt sein kann, oder C₃₋₇-Cycloalkyl ist;
R⁵ NO₂, F, CF₃, SO₂NR''R''', worin R'' und R''' unabhängig voneinander Wasserstoff oder C₁₋₆-Alkyl sind, oder CN ist; und
R⁴, R⁶, R⁷ unabhängig voneinander Wasserstoff, C₁₋₆-Alkyl, welches verzweigt sein kann, Phenyl, Halogen, C₁₋₆-Alkoxy, NO₂, CN, SO₂NR''R''', worin R'' und R''' unabhängig voneinander Wasserstoff oder C₁₋₆-Alkyl sind, oder CF₃ sind, oder R⁶ und R⁷ zusammen einen zusätzlichen 4- bis 7-gliedrigen Ring bilden, welcher aromatisch oder teilweise gesättigt sein kann und welcher mit Halogen, NO₂, CF₃, CN, SO₂NR''R''', worin R'' und R''' unabhängig voneinander Wasserstoff oder C₁₋₆-Alkyl sind, substituiert sein kann, und R⁴ die vorstehend angegebene Bedeutung hat, zur Herstellung eines Arzeimittels, das bei der Behandlung von Zuständen, die auf eine excitatorische Aminosäure ansprechen, brauchbar ist.

10. Verwendung nach Anspruch 9, worin mindestens eines von R⁴, R⁵, R⁶ und R⁷ NO₂, CF₃, CN, SO₂NR''R''' oder Halogen ist und R¹, R², R⁴, R⁵, R⁶, R⁷, R'' und R''' im übrigen die im Anspruch 1 angegebenen Bedeutungen haben.

11. Verwendung nach Anspruch 9, worin R⁵ NO₂, F, CF₃ oder CN ist.

## Patentansprüche (Patentansprüche für folgende(n) Vertragsstaat(en): ES)

1. Verfahren zum Herstellen einer Verbindung mit der Formel 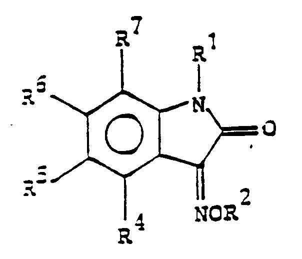 worin R¹ Wasserstoff, C₁₋₆-Alkyl, welches verzweigt sein kann, C₃₋₇-Cycloalkyl, Benzyl, Acyl, Hydroxy, C₁₋₆-Alkoxy, CH₂CO₂R', worin R' Wasserstoff oder C₁₋₆-Alkyl, welches verzweigt sein kann, ist, CH₂CN, CH₂CONR^{IV}R^{V}, worin R^{IV} und R^{V} unabhängig voneinander Wasserstoff oder C₁₋₆-Alkyl sind, oder CH₂C(=NOH)NH₂ ist;
R² Wasserstoff, Benzyl, C₁₋₆-Alkyl, welches verzweigt sein kann, oder C₃₋₇-Cycloalkyl ist;
R⁵ NO₂, F, CF₃, SO₂NR''R''', worin R'' und R''' unabhängig voneinander Wasserstoff oder C₁₋₆-Alkyl sind, oder CN ist; und R⁴, R⁶, R⁷ unabhängig voneinander Wasserstoff, C₁₋₆-Alkyl, welches verzweigt sein kann, Phenyl, Halogen, C₁₋₆-Alkoxy, NO₂, CN, SO₂NR''R''', worin R'' und R''' unabhängig voneinander Wasserstoff oder C₁₋₆-Alkyl sind, oder CF₃ sind, oder R⁶ und R⁷ zusammen einen zusätzlichen 4- bis 7-gliedrigen Ring bilden, welcher aromatisch oder teilweise gesättigt sein kann und welcher mit Halogen, NO₂, CF₃, CN, SO₂NR''R''', worin R'' und R''' unabhängig voneinander Wasserstoff oder C₁₋₆-Alkyl sind, substituiert sein kann, und R⁴ die vorstehend angegebene Bedeutung hat, mit der Maßgabe, daß R⁵ von NO₂ und F verschieden ist, wenn R¹, R⁴, R⁶ und R⁷ Wasserstoff sind, und R² Wasserstoff oder Benzyl ist; und daß R⁵ von NO₂ verschieden ist, wenn R¹, R², R⁴ und R⁶ Wasserstoff sind und R⁷ NO₂ ist, umfassend den Schritt des Umsetzens einer Verbindung der Formel 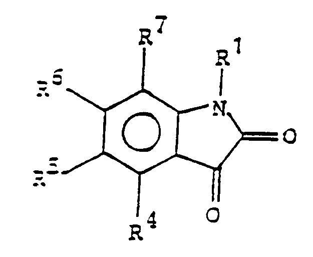 worin R¹, R⁴, R⁵, R⁶ und R⁷ die vorstehend angegebene Bedeutung haben, mit einer Verbindung der Formel NH₂OR², worin R² die vorstehend angegebene Bedeutung hat.

2. Verfahren nach Anspruch 1, worin R⁵ F, NO₂, CN, CF₃ oder SO₂NR''R''', worin R'' und R''' unabhängig voneinander Wasserstoff oder C₁₋₆-Alkyl sind, ist, und worin R⁶ und R⁷ zusammen einen zusätzlichen 4- bis 7-gliedrigen Ring bilden, welcher aromatisch oder teilweise gesättigt sein kann und welcher mit Halogen, NO₂, CF₃, CN, SO₂NR''R''', worin R'' und R''' unabhängig voneinander Wasserstoff oder C₁₋₆-Alkyl sind, substituiert sein kann.

3. Verfahren nach Anspruch 2, worin der zusätzliche Ring, der durch R⁶ und R⁷ gebildet wird, mit Halogen, NO₂, CF₃, CN, oder SO₂NR''R''' substituiert ist, worin R'' und R''' unabhängig voneinander Wasserstoff oder C₁₋₆-Alkyl sind.

4. Verfahren nach Anspruch 1, worin die hergestellte Verbindung 5-Nitro-1H-benz[g]indol-2,3-dion-3-oxim ist.

5. Verfahren nach Anspruch 1, worin die hergestellte Verbindung 5,7-Dinitro-1-methyl-1H-indol-2,3-dion-3-(O-methyloxim) ist.

6. Verfahren nach Anspruch 1, worin die hergestellte Verbindung 5-Nitro-1H-6,7,8,9-tetrahydro-benz[g]indol-2,3-dion-3-oxim ist.

7. Verfahren zum Herstellen einer pharmazeutischen Zusammensetzung, umfassend eine wirksame excitatorischen Aminosäuren entgegenwirkende Menge einer Verbindung mit der Formel 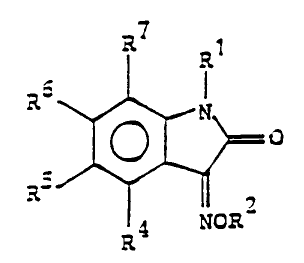 worin R¹ Wasserstoff, C₁₋₆-Alkyl, welches verzweigt sein kann, C₃₋₇-Cycloalkyl, Benzyl, Acyl, Hydroxy, C₁₋₆-Alkoxy, CH₂CO₂R', worin R' Wasserstoff oder C₁₋₆-Alkyl, welches verzweigt sein kann, ist, CH₂CN, CH₂CONR^{IV}R^{V}, worin R^{IV} und R^{V} unabhängig voneinander Wasserstoff oder C₁₋₆-Alkyl sind, oder CH₂C(=NOH)NH₂ ist;
R² Wasserstoff, Benzyl, C₁₋₆-Alkyl, welches verzweigt sein kann, oder C₃₋₇-Cycloalkyl ist;
R⁵ NO₂, F, CF₃, SO₂NR''R''', worin R'' und R''' unabhängig voneinander Wasserstoff oder C₁₋₆-Alkyl sind, oder CN ist; und R⁴, R⁶, R⁷ unabhängig voneinander Wasserstoff, C₁₋₆-Alkyl, welches verzweigt sein kann, Phenyl, Halogen, C₁₋₆-Alkoxy, NO₂, CN, SO₂NR''R''', worin R'' und R''' unabhängig voneinander Wasserstoff oder C₁₋₆-Alkyl sind, oder CF₃ sind, oder R⁶ und R⁷ zusammen einen zusätzlichen 4- bis 7-gliedrigen Ring bilden, welcher aromatisch oder teilweise gesättigt sein kann und welcher mit Halogen, NO₂, CF₃, CN, SO₂NR''R''', worin R'' und R''' unabhängig voneinander Wasserstoff oder C₁₋₆-Alkyl sind, substituiert sein kann, und R⁴ die vorstehend angegebene Bedeutung hat, durch Kombinieren der Verbindung mit herkömmlichen Additiven in einer an sich bekannten Weise.

## Patentansprüche (Patentansprüche für folgende(n) Vertragsstaat(en): GR)

1. Verbindung mit der Formel 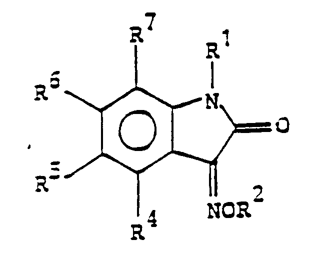 worin R¹ Wasserstoff, C₁₋₆-Alkyl, welches verzweigt sein kann, C₃₋₇-Cycloalkyl, Benzyl, Acyl, Hydroxy, C₁₋₆-Alkoxy, CH₂CO₂R', worin R' Wasserstoff oder C₁₋₆-Alkyl, welches verzweigt sein kann, ist, CH₂CN, CH₂CONR^{IV}R^{V}, worin R^{IV} und R^{V} unabhängig voneinander Wasserstoff oder C₁₋₆-Alkyl sind, oder CH₂C(=NOH)NH₂ ist;
R² Wasserstoff, Benzyl, C₁₋₆-Alkyl, welches verzweigt sein kann, oder C₃₋₇-Cycloalkyl ist;
R⁵ NO₂, F, CF₃, SO₂NR''R''', worin R'' und R''' unabhängig voneinander Wasserstoff oder C₁₋₆-Alkyl sind, oder CN ist; und R⁴, R⁶, R⁷ unabhängig voneinander Wasserstoff, C₁₋₆-Alkyl, welches verzweigt sein kann, Phenyl, Halogen, C₁₋₆-Alkoxy, NO₂, CN, SO₂NR''R''', worin R'' und R''' unabhängig voneinander Wasserstoff oder C₁₋₆-Alkyl sind, oder CF₃ sind, oder R⁶ und R⁷ zusammen einen zusätzlichen 4- bis 7-gliedrigen Ring bilden, welcher aromatisch oder teilweise gesättigt sein kann und welcher mit Halogen, NO₂, CF₃, CN, SO₂NR''R''', worin R'' und R''' unabhängig voneinander Wasserstoff oder C₁₋₆-Alkyl sind, substituiert sein kann, und R⁴ die vorstehend angegebene Bedeutung hat, mit der Maßgabe, daß R⁵ von NO₂ und F verschieden ist, wenn R¹, R⁴, R⁶ und R⁷ Wasserstoff sind, und R² Wasserstoff oder Benzyl ist; und daß R⁵ von NO₂ verschieden ist, wenn R¹, R², R⁴ und R⁶ Wasserstoff sind und R⁷ NO₂ ist.

2. Verbindung nach Anspruch 1, worin R⁵ F, NO₂, CN, CF₃ oder SO₂NR''R''', worin R'' und R''' unabhängig voneinander Wasserstoff oder C₁₋₆-Alkyl sind, ist, und worin R⁶ und R⁷ zusammen einen zusätzlichen 4- bis 7-gliedrigen Ring bilden, welcher aromatisch oder teilweise gesättigt sein kann und welcher mit Halogen, NO₂, CF₃, CN, SO₂NR''R''', worin R'' und R''' unabhängig voneinander Wasserstoff oder C₁₋₆-Alkyl sind, substituiert sein kann.

3. Verbindung nach Anspruch 2, worin der zusätzliche Ring, der durch R⁶ und R⁷ gebildet wird, mit Halogen, NO₂, CF₃, CN, oder SO₂NR''R''' substituiert ist, worin R'' und R''' unabhängig voneinander Wasserstoff oder C₁₋₆-Alkyl sind.

4. Verbindung nach Anspruch 1, welche 5-Nitro-1H-benz[g]indol-2,3-dion-3-oxim ist.

5. Verbindung nach Anspruch 1, welche 5,7-Dinitro-1-methyl-1H-indol-2,3-dion-3-(O-methyloxim) ist.

6. Verbindung nach Anspruch 1, welche 5-Nitro-1H-6,7,8,9-tetrahydro-benz[g]indol-2,3-dion-3-oxim ist.

7. Verfahren zum Herstellen einer pharmazeutischen Zusammensetzung, umfassend eine wirksame excitatorischen Aminosäuren entgegenwirkende Menge einer Verbindung mit der Formel 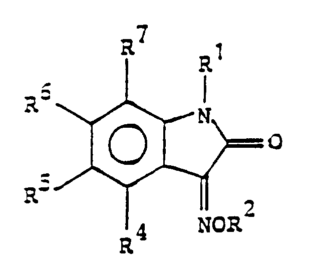 worin R¹ Wasserstoff, C₁₋₆-Alkyl, welches verzweigt sein kann, C₃₋₇-Cycloalkyl, Benzyl, Acyl, Hydroxy, C₁₋₆-Alkoxy, CH₂CO₂R', worin R' Wasserstoff oder C₁₋₆-Alkyl, welches verzweigt sein kann, ist, CH₂CN, CH₂CONR^{IV}R^{V}, worin R^{IV} und R^{V} unabhängig voneinander Wasserstoff oder C₁₋₆-Alkyl sind, oder CH₂C(=NOH)NH₂ ist;
R² Wasserstoff, Benzyl, C₁₋₆-Alkyl, welches verzweigt sein kann, oder C₃₋₇-Cycloalkyl ist;
R⁵ NO₂, F, CF₃, SO₂NR''R''', worin R'' und R''' unabhängig voneinander Wasserstoff oder C₁₋₆-Alkyl sind, oder CN ist; und R⁴, R⁶, R⁷ unabhängig voneinander Wasserstoff, C₁₋₆-Alkyl, welches verzweigt sein kann, Phenyl, Halogen, C₁₋₆-Alkoxy, NO₂, CN, SO₂NR''R''', worin R'' und R''' unabhängig voneinander Wasserstoff oder C₁₋ ₆-Alkyl sind, oder CF₃ sind, oder R⁶ und R⁷ zusammen einen zusätzlichen 4- bis 7-gliedrigen Ring bilden, welcher aromatisch oder teilweise gesättigt sein kann und welcher mit Halogen, NO₂, CF₃, CN, SO₂NR''R''', worin R'' und R''' unabhängig voneinander Wasserstoff oder C₁₋ ₆-Alkyl sind, substituiert sein kann, und R⁴ die vorstehend angegebene Bedeutung hat, durch Kombinieren der Verbindung mit herkömmlichen Additiven in einer an sich bekannten Weise.

8. Verfahren zum Herstellen der Verbindung nach Anspruch 1, umfassend den Schritt des Umsetzens einer Verbindung der Formel 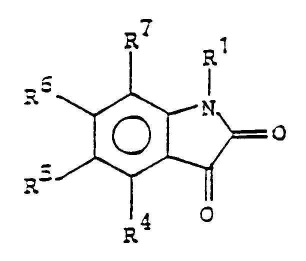 worin R¹, R⁴, R⁵, R⁶ und R⁷ die in Anspruch 1 angegebene Bedeutung haben, mit einer Verbindung mit der Formel NH₂OR², worin R² die in Anspruch 1 angegebene Bedeutung hat.

9. Verwendung einer Indol-2,3-dion-3-oxim-Verbindung mit der Formel 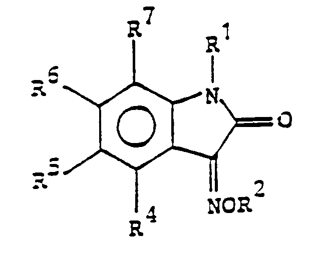 worin R¹ Wasserstoff, C₁-C₆-Alkyl, welches verzweigt sein kann, C₃₋₇-Cycloalkyl, Benzyl, Phenyl, Acyl, Hydroxy, C₁₋ ₆-Alkoxy, CH₂CO₂R', worin R' Wasserstoff oder C₁₋₆-Alkyl, welches verzweigt sein kann, ist, CH₂CN, CH₂CONR^{IV}R^{V}, worin R^{IV} und R^{V} unabhängig voneinander Wasserstoff oder C₁₋₆-Alkyl sind, oder CH₂C(=NOH)NH₂ ist;
R² Wasserstoff, Benzyl, C₁₋₆-Alkyl, welches verzweigt sein kann, oder C₃₋₇-Cycloalkyl ist;
R⁵ NO₂, F, CF₃, SO₂NR''R''', worin R'' und R''' unabhängig voneinander Wasserstoff oder C₁₋₆-Alkyl sind, oder CN ist; und R⁴, R⁶, R⁷ unabhängig voneinander Wasserstoff, C₁₋₆-Alkyl, welches verzweigt sein kann, Phenyl, Halogen, C₁₋₆-Alkoxy, NO₂, CN, SO₂NR''R''', worin R'' und R''' unabhängig voneinander Wasserstoff oder C₁₋ ₆-Alkyl sind, oder CF₃ sind, oder R⁶ und R⁷ zusammen einen zusätzlichen 4- bis 7-gliedrigen Ring bilden, welcher aromatisch oder teilweise gesättigt sein kann und welcher mit Halogen, NO₂, CF₃, CN, SO₂NR''R''', worin R'' und R''' unabhängig voneinander Wasserstoff oder C₁₋ ₆-Alkyl sind, substituiert sein kann, und R⁴ die vorstehend angegebene Bedeutung hat, zur Herstellung eines Arzeimittels, das bei der Behandlung von Zuständen, die auf eine excitatorische Aminosäure ansprechen, brauchbar ist.

10. Verwendung nach Anspruch 9, worin mindestens eines von R⁴, R⁵, R⁶ und R⁷ NO₂, CF₃, CN, SO₂NR''R''' oder Halogen ist und R¹, R², R⁴, R⁵, R⁶, R⁷, R'' und R''' im übrigen die in Anspruch 1 angegebenen Bedeutungen haben.

11. Verwendung nach Anspruch 9, worin R⁵ NO₂, F, CF₃ oder CN ist.

## Revendications (Revendications pour l'(les) Etat(s) contractant(s) suivant(s): AT, BE, CH, LI, DE, DK, FR, GB, IT, LU, NL, SE)

1. Composé de formule : 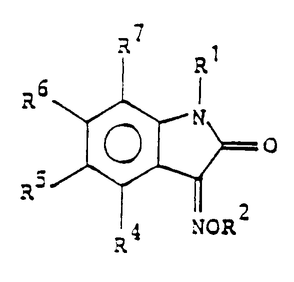 dans laquelle R¹ représente un atome d'hydrogène, un groupe alkyle en C₁-C₆ qui peut être ramifié, cycloalkyle en C₃-C₇, benzyle, acyle, hydroxy, alkoxy en C₁-C₆, CH₂CO₂R' dans lequel R' représente un atome d'hydrogène ou un groupe alkyle en C₁-C₆ qui peut être ramifié, CH₂CN, CH₂CONR^{IV}R^{V} dans lequel R^{IV} et R^{V} représentent indépendamment un atome d'hydrogène ou un groupe alkyle en C₁-C₆, ou CH₂C(=NOH)NH₂ ;
R² représente un atome d'hydrogène, un groupe benzyle, alkyle en C₁-C₆ qui peut être ramifié ou cycloalkyle en C₃-C₇ ;
R⁵ représente NO₂, F, CF₃, SO₂NR''R''', R'' et R''' représentant indépendamment un atome d'hydrogène ou un groupe alkyle en C₁-C₆, ou CN ; et
R⁴, R⁶ et R⁷ représentent indépendamment un atome d'hydrogène, un groupe alkyle en C₁-C₆ qui peut être ramifié, un groupe phényle, un halogène, un groupe alkoxy en C₁-C₆, un groupe NO₂, un groupe CN, un groupe SO₂NR''R''' dans lequel R'' et R''' représentent indépendamment un atome d'hydrogène ou un groupe alkyle en C₁-C₆, ou un groupe CF₃, ou R⁶ et R⁷ forment ensemble un cycle supplémentaire de 4 à 7 chaînons qui peut être aromatique ou partiellement saturé, et qui peut être substitué avec un halogène, un groupe NO₂, CF₃, CN, SO₂NR''R''' dans lequel R'' et R''' représentent indépendamment un atome d'hydrogène ou un groupe alkyle en C₁-C₆, et R⁴ a la même définition que ci-dessus, à condition que R⁵ soit différent de NO₂ et F lorsque R¹, R⁴, R⁶ et R⁷ représentent un atome d'hydrogène, et R² représente un atome d'hydrogène ou un groupe benzyle ; et que R⁵ soit différent de NO₂ lorsque R¹, R², R⁴ et R⁶ représentent un atome d'hydrogène et R⁷ représente NO₂.

2. Composé selon la revendication 1, dans lequel R⁵ représente F, NO₂, CN, CF₃ ou SO₂NR''R''', R'' et R''' représentant indépendamment un atome d'hydrogène ou un groupe alkyle en C₁-C₆, et R⁶ et R⁷ forment ensemble un cycle supplémentaire de 4 à 7 chaînons qui peut être aromatlque ou partiellement saturé, et qui peut être substitué avec un atome d'halogène, un groupe NO₂, CF₃, CN, SO₂NR''R''', R'' et R''' représentant indépendamment un atome d'hydrogène ou un groupe alkyle en C₁-C₆.

3. Composé selon la revendication 2, dans lequel le cycle supplémentaire formé par R⁶ et R⁷, est substitué avec un halogène, un groupe NO₂, CF₃, CN ou SO₂NR''R''', R'' et R''' représentant indépendamment un atome d'hydrogène ou un groupe alkyle en C₁-C₆.

4. Composé selon la revendication 1, qui est la 5-nitro-1H-benz[g]-indole-2,3-dione-3-oxime.

5. Composé selon la revendication 1, qui est la 5,7-dinitro-1-méthyl-1H-indole-2,3-dione-3-(O-méthyloxime).

6. Composé selon la revendication 1, qui est la 5-nitro-1H-6,7,8,9-tétrahydro-benz[g]indole-2,3-dione-3-oxime.

7. Composition pharmaceutique comprenant une quantité antagoniste efficace à l'égard d'un aminoacide excitant, d'un composé de formule : 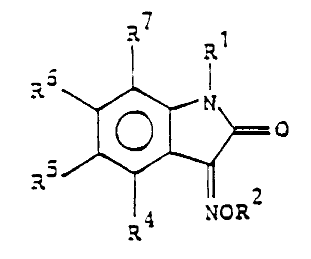 dans laquelle R¹ représente un atome d'hydrogène, un groupe alkyle en C₁-C₆ qui peut être ramifié, un groupe cycloalkyle en C₃-C₇, benzyle, acyle, hydroxy, alkoxy en C₁-C₆, CH₂CO₂R' dans lequel R' représente un atome d'hydrogène ou un groupe alkyle en C₁-C₆ qui peut être ramifié, CH₂CN, CH₂CONR^{IV}R^{V} dans lequel R^{IV} et R^{V} représentent indépendamment un atome d'hydrogène ou un groupe alkyle en C₁-C₆, ou CH₂C(=NOH)NH₂ ;
R² représente un atome d'hydrogène, un groupe benzyle, un groupe alkyle en C₁-C₆ qui peut être ramifié ou cycloalkyle en C₃-C₇ ;
R⁵ représente NO₂, F, CF₃, SO₂NR''R''', R'' et R''' représentant indépendamment un atome d'hydrogène ou un groupe alkyle en C₁-C₆, ou CN ; et
R⁴, R⁶, R⁷ représentent indépendamment un atome d'hydrogène, un groupe alkyle en C₁-C₆ qui peut être ramifié, un groupe phényle, un halogène, un groupe alkoxy en C₁-C₆, un groupe NO₂, un groupe CN, un groupe SO₂NR''R''' dans lequel R'' et R''' représentent indépendamment un atome d'hydrogène ou un groupe alkyle en C₁-C₆, ou un groupe CF₃, ou R⁶ et R⁷ forment ensemble un cycle supplémentaire de 4 à 7 chaînons qui peut être aromatique ou partiellement saturé, et qui peut être substitué avec un halogène, un groupe NO₂, CF₃, CN, SO₂NR''R''' dans lequel R'' et R''' représentent indépendamment un atome d'hydrogène ou un groupe alkyle en C₁-C₆, et R⁴ a la même définition que ci-dessus.

8. Procédé de préparation du composé selon la revendication 1, comprenant l'opération consistant à faire réagir un composé de formule : 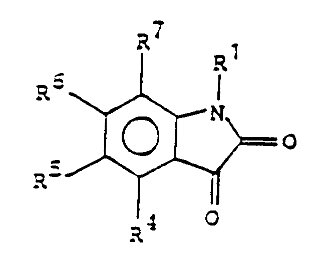 dans laquelle R¹, R⁴, R⁵, R⁶ et R⁷ ont les mêmes définitions que celles mentionnées dans la revendication 1, avec un composé de formule NH₂OR² dans laquelle R² a la même définition que dans la revendication 1.

9. Utilisation d'un composé d'indole-2,3-dione-3-oxime de formule : 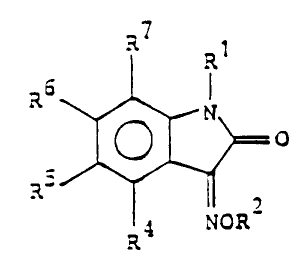 dans laquelle :
R¹ représente un atome d'hydrogène, un groupe alkyle en C₁-C₆ qui peut être ramifié, cycloalkyle en C₃-C₇, benzyle, phényle, acyle, hydroxy, alkoxy en C₁-C₆, CH₂CO₂R' dans lequel R' représente un atome d'hydrogène ou un groupe alkyle en C₁-C₆ qui peut être ramifié, CH₂CN, CH₂CONR^{IV}R^{V} dans lequel R^{IV} et R^{V} représentent indépendamment un atome d'hydrogène ou un groupe alkyle en C₁-C₆, ou CH₂C(=NOH)NH₂ ;
R² représente un atome d'hydrogène, un groupe benzyle, alkyle en C₁-C₆ qui peut être ramifié, ou cycloalkyle en C₃-C₇ ;
R⁵ représente NO₂, F, CF₃, SO₂NR''R''', R'' et R''' représentant indépendamment un atome d'hydrogène ou un groupe alkyle en C₁-C₆, ou CN ; et
R⁴, R⁶, R⁷ représentent indépendamment un atome d'hydrogène, un groupe alkyle en C₁-C₆ qui peut être ramifié, un groupe phényle, un halogène, un groupe alkoxy en C₁-C₆, NO₂, CN, SO₂NR''R''', R'' et R''' représentant indépendamment un atome d'hydrogène ou un groupe alkyle en C₁-C₆, ou un groupe CF₃, ou R⁶ et R⁷ forment ensemble un cycle supplémentaire de 4 à 7 chaînons qui peut être aromatique ou partiellement saturé, et qui peut être substitué avec un halogène, un groupe NO₂, CF₃, CN, SO₂NR''R''', R'' et R''' représentant indépendamment un atome d'hydrogène ou un groupe alkyle en C₁-C₆, et R⁴ a la même définition que ci-dessus, pour la préparation d'un médicament utile pour le traitement d'affections sensibles à un aminoacide excitant.

10. Utilisation selon la revendication 9, dans laquelle au moins l'un des groupes R⁴, R⁵, R⁶ et R⁷, représente NO₂, CF₃, CN, SO₂NR''R''' ou un halogène, et R¹, R², R⁴, R⁵, R⁶, R⁷, R'' et R''' ont autrement les mêmes définitions que dans la revendication 1.

11. Utilisation selon la revendication 9, dans laquelle R⁵ représente NO₂, F, CF₃ ou CN.

## Revendications (Revendications pour l'(les) Etat(s) contractant(s) suivant(s): ES)

1. Procédé de préparation d'un composé de formule : 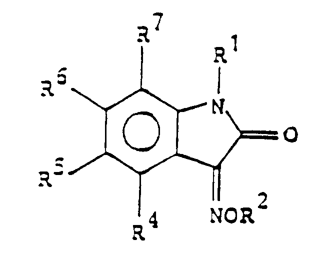 dans laquelle R¹ représente un atome d'hydrogène, un groupe alkyle en C₁-C₆ qui peut être ramifié, cycloalkyle en C₃-C₇, benzyle, acyle, hydroxy, alkoxy en C₁-C₆, CH₂CO₂R' dans lequel R' représente un atome d'hydrogène ou un groupe alkyle en C₁-C₆ qui peut être ramifié, CH₂CN, CH₂CONR^{IV}R^{V} dans lequel R^{IV} et R^{V} représentent indépendamment un atome d'hydrogène ou un groupe alkyle en C₁-C₆ ou CH₂C(=NOH)NH₂ ;
R² représente un atome d'hydrogène, un groupe benzyle, alkyle en C₁-C₆ qui peut être ramifié ou cycloalkyle en C₃-C₇ ;
R⁵ représente NO₂, F, CF₃, SO₂NR''R''', R'' et R''' représentant indépendamment un atome d'hydrogène ou un groupe alkyle en C₁-C₆, ou CN ; et R⁴, R⁶ et R⁷ représentent indépendamment un atome d'hydrogène, un groupe alkyle en C₁-C₆ qui peut être ramifié, un groupe phényle, un halogène, un groupe alkoxy en C₁-C₆, un groupe NO₂, CN, SO₂NR''R''', R'' et R''' représentant indépendamment un atome d'hydrogène ou un groupe alkyle en C₁-C₆, ou un groupe CF₃, ou R⁶ et R⁷ forment ensemble un cycle supplémentaire de 4 à 7 chaînons qui peut être aromatique ou partiellement saturé, et qui peut être substitué avec un halogène, un groupe NO₂, CF₃, CN, SO₂NR''R''', R'' et R''' représentant indépendamment un atome d'hydrogène ou un groupe alkyle en C₁-C₆, et R⁴ a la même définition que ci-dessus, à condition que R⁵ soit différent de NO₂ et F lorsque R¹, R⁴, R⁶ et R⁷ représentent un atome d'hydrogène, et R² représente un atome d'hydrogène ou un groupe benzyle ; et que R⁵ soit différent de NO₂ lorsque R¹, R², R⁴ et R⁶ représentent un atome d'hydrogène et R⁷ représente NO₂, comprenant les étapes consistant à faire réagir un composé de formule : 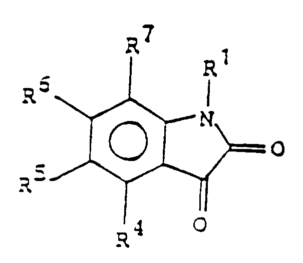 dans laquelle R¹, R⁴, R⁵, R⁶ et R⁷ ont la même définition que ci-dessus, avec un composé de formule NH₂OR² dans laquelle R² a la même définition que ci-dessus.

2. Procédé selon la revendication 1, dans lequel R⁵ représente F, NO₂, CN, CF₃ ou SO₂NR''R''', R'' et R''' représentant indépendamment un atome d'hydrogène ou un groupe alkyle en C₁-C₆, et R⁶ et R⁷ forment ensemble un cycle supplémentaire de 4 à 7 chaînons qui peut être aromatique ou partiellement saturé, et qui peut être substitué avec un halogène, un groupe NO₂, CF₃, CN, SO₂NR''R''', R'' et R''' représentant indépendamment un atome d'hydrogène ou un groupe alkyle en C₁-C₆.

3. Procédé selon la revendication 2, dans lequel le cycle supplémentaire formé par R⁶ et R⁷, est substitué avec un halogène, un groupe NO₂, CF₃, CN ou SO₂NR''R''', R'' et R''' représentant indépendamment un atome d'hydrogène ou un groupe alkyle en C₁-C₆.

4. Procédé selon la revendication 1, dans lequel le composé préparé est la 5-nitro-1H-benz[g]-indole-2,3-dione-3-oxime.

5. Procédé selon la revendication 1, dans lequel le composé préparé est la 5,7-dinitro-1-méthyl-1H-indole-2,3-dione-3-(O-méthyloxime).

6. Procédé selon la revendication 1, dans lequel le composé préparé est la 5-nitro-1H-6,7,8,9-tétrahydro-benz[g]indole-2,3-dione-3-oxime.

7. Procédé de préparation d'une composition pharmaceutique comprenant une quantité antagoniste efficace à l'égard d'un aminoacide excitant, d'un composé de formule : 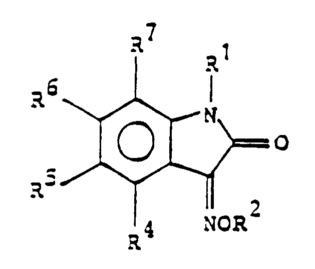 dans laquelle R¹ représente un atome d'hydrogène, un groupe alkyle en C₁-C₆ qui peut être ramifié, un groupe cycloalkyle en C₃-C₇, benzyle, acyle, hydroxy, alkoxy en C₁-C₆, CH₂CO₂R', R' représentant un atome d'hydrogène ou un groupe alkyle en C₁-C₆ qui peut être ramifié, CH₂CN, CH₂CONR^{IV}R^{V}, R^{IV} et R^{V} représentant indépendamment un atome d'hydrogène ou un groupe alkyle en C₁-C₆, ou un groupe CH₂C(=NOH)NH₂ ;
R² représente un atome d'hydrogène, un groupe benzyle, alkyle en C₁-C₆ qui peut être ramifié, ou un groupe cycloalkyle en C₃-C₇ ;
R⁵ représente NO₂, F, CF₃, SO₂NR''R''' dans lequel R'' et R''' représentent indépendamment un atome d'hydrogène ou un groupe alkyle en C₁-C₆, ou CN ; et R⁴, R⁶ et R⁷ représentent indépendamment un atome d'hydrogène, un groupe alkyle en C₁-C₆ qui peut être ramifié, un groupe phényle, un halogène, un groupe alkoxy en C₁-C₆, NO₂, CN, SO₂NR''R''', R'' et R''' représentant indépendamment un atome d'hydrogène ou un groupe alkyle en C₁-C₆, ou un groupe CF₃, ou R⁶ et R⁷ forment ensemble un cycle supplémentaire de 4 à 7 chaînons qui peut être aromatique ou partiellement saturé et qui peut être substitué avec un halogène, un groupe NO₂, CF₃, CN, SO₂NR''R''', R'' et R''' représentant indépendamment un atome d'hydrogène ou un groupe alkyle en C₁-C₆, et R⁴ a la même définition que ci-dessus, en associant le composé avec des additifs classiques de façon connue en soi.

## Revendications (Revendications pour l'(les) Etat(s) contractant(s) suivant(s): GR)

1. Composé de formule : 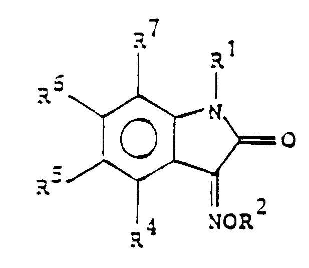 dans laquelle R¹ représente un atome d'hydrogène, un groupe alkyle en C₁-C₆ qui peut être ramifié, cycloalkyle en C₃-C₇, benzyle, acyle, hydroxy, alkoxy en C₁-C₆, CH₂CO₂R' dans lequel R' représente un atome d'hydrogène ou un groupe alkyle en C₁-C₆ qui peut être ramifié, CH₂CN, CH₂CONR^{IV}R^{V} dans lequel R^{IV} et R^{V} représentent indépendamment un atome d'hydrogène ou un groupe alkyle en C₁-C₆, ou CH₂C(=NOH)NH₂ ;
R² représente un atome d'hydrogène, un groupe benzyle, alkyle en C₁-C₆ qui peut être ramifié ou cycloalkyle en C₃-C₇ ;
R⁵ représente NO₂, F, CF₃, SO₂NR''R''', R'' et R''' représentant indépendamment un atome d'hydrogène ou un groupe alkyle en C₁-C₆, ou CN ; et
R⁴, R⁶ et R⁷ représentent indépendamment un atome d'hydrogène, un groupe alkyle en C₁-C₆ qui peut être ramifié, un groupe phényle, un halogène, un groupe alkoxy en C₁-C₆, un groupe NO₂, un groupe CN, un groupe SO₂NR''R''' dans lequel R'' et R''' représentent indépendamment un atome d'hydrogène ou un groupe alkyle en C₁-C₆, ou un groupe CF₃, ou R⁶ et R⁷ forment ensemble un cycle supplémentaire de 4 à 7 chaînons qui peut être aromatique ou partiellement saturé, et qui peut être substitué avec un halogène, un groupe NO₂, CF₃, CN, SO₂NR''R''' dans lequel R'' et R''' représentent indépendamment un atome d'hydrogène ou un groupe alkyle en C₁-C₆, et R⁴ a la même définition que ci-dessus, à condition que R⁵ soit différent de NO₂ et F lorsque R¹, R⁴, R⁶ et R⁷ représentent un atome d'hydrogène, et R² représente un atome d'hydrogène ou un groupe benzyle ; et que R⁵ soit différent de NO₂ lorsque R¹, R², R⁴ et R⁶ représentent un atome d'hydrogène et R⁷ représente NO₂.

2. Composé selon la revendication 1, dans lequel R⁵ représente F, NO₂, CN, CF₃ ou SO₂NR''R''', R'' et R''' représentant indépendamment un atome d'hydrogène ou un groupe alkyle en C₁-C₆, et R⁶ et R⁷ forment ensemble un cycle supplémentaire de 4 à 7 chaînons qui peut être aromatique ou partiellement saturé, et qui peut être substitué avec un atome d'halogène, un groupe NO₂, CF₃, CN, SO₂NR''R''', R'' et R''' représentant indépendamment un atome d'hydrogène ou un groupe alkyle en C₁-C₆.

3. Composé selon la revendication 2, dans lequel le cycle supplémentaire formé par R⁶ et R⁷, est substitué avec un halogène, un groupe NO₂, CF₃, CN ou SO₂NR''R''', R'' et R''' représentant indépendamment un atome d'hydrogène ou un groupe alkyle en C₁-C₆.

4. Composé selon la revendication 1, qui est la 5-nitro-1H-benz[g]-indole-2,3-dione-3-oxime.

5. Composé selon la revendication 1, qui est la 5,7-dinitro-1-méthyl-1H-indole-2,3-dione-3-(O-méthyloxime).

6. Composé selon la revendication 1, qui est la 5-nitro-1H-6,7,8,9-tétrahydro-benz[g]indole-2,3-dione-3-oxime.

7. Procédé de préparation d'une composition pharmaceutique comprenant une quantité antagoniste efficace à l'égard d'un aminoacide excitant, d'un composé de formule : 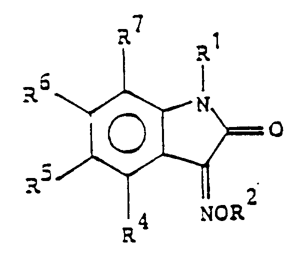 dans laquelle R¹ représente un atome d'hydrogène, un groupe alkyle en C₁-C₆ qui peut être ramifié, un groupe cycloalkyle en C₃-C₇, benzyle, acyle, hydroxy, alkoxy en C₁-C₆, CH₂CO₂R', R' représentant un atome d'hydrogène où un groupe alkyle en C₁-C₆ qui peut être ramifié, CH₂CN, CH₂CONR^{IV}R^{V}, R^{IV} et R^{V} représentant indépendamment un atome d'hydrogène ou un groupe alkyle en C₁-C₆, ou un groupe CH₂C(=NOH)NH₂ ;
R² représente un atome d'hydrogène, un groupe benzyle, alkyle en C₁-C₆ qui peut être ramifié, ou un groupe cycloalkyle en C₃-C₇ ;
R⁵ représente NO₂, F, CF₃, SO₂NR''R''' dans lequel R'' et R''' représentent indépendamment un atome d'hydrogène ou un groupe alkyle en C₁-C₆, ou CN ; et R⁴, R⁶ et R⁷ représentent indépendamment un atome d'hydrogène, un groupe alkyle en C₁-C₆ qui peut être ramifié, un groupe phényle, un halogène, un groupe alkoxy en C₁-C₆, NO₂, CN, SO₂NR''R''', R'' et R''' représentant indépendamment un atome d'hydrogène ou un groupe alkyle en C₁-C₆, ou un groupe CF₃, ou R⁶ et R⁷ forment ensemble un cycle supplémentaire de 4 à 7 chaînons qui peut être aromatique ou partiellement saturé et qui peut être substitué avec un halogène, un groupe NO₂, CF₃, CN, SO₂NR''R''', R'' et R''' représentant indépendamment un atome d'hydrogène ou un groupe alkyle en C₁-C₆, et R⁴ a la même définition que ci-dessus, en associant le composé avec des additifs classiques de façon connue en soi.

8. Procédé de préparation du composé selon la revendication 1, comprenant l'opération consistant à faire réagir un composé de formule : 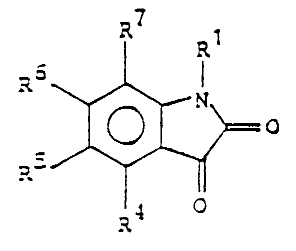 dans laquelle R¹, R⁴, R⁵, R⁶ et R⁷ ont les mêmes définitions que celles mentionnées dans la revendication 1, avec un composé de formule NH₂OR² dans laquelle R² a la même définition que dans la revendication 1.

9. Utilisation d'un composé d'indole-2,3-dione-3-oxime de formule : 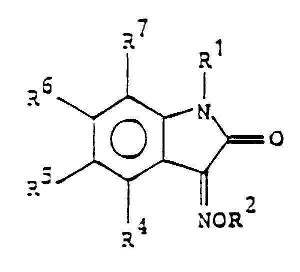 dans laquelle :
R¹ représente un atome d'hydrogène, un groupe alkyle en C₁-C₆ qui peut être ramifié, cycloalkyle en C₃-C₇, benzyle, phényle, acyle, hydroxy, alkoxy en C₁-C₆, CH₂CO₂R' dans lequel R' représente un atome d'hydrogène ou un groupe alkyle en C₁-C₆ qui peut être ramifié, CH₂CN, CH₂CONR^{IV}R^{V} dans lequel R^{IV} et R^{V} représentent indépendamment un atome d'hydrogène ou un groupe alkyle en C₁-C₆, ou CH₂C(=NOH)NH₂ ;
R² représente un atome d'hydrogène, un groupe benzyle, alkyle en C₁-C₆ qui peut être ramifié, ou cycloalkyle en C₃-C₇ ;
R⁵ représente NO₂, F, CF₃, SO₂NR''R''', R'' et R''' représentant indépendamment un atome d'hydrogène ou un groupe alkyle en C₁-C₆, ou CN ; et
R⁴, R⁶, R⁷ représentent indépendamment un atome d'hydrogène, un groupe alkyle en C₁-C₆ qui peut être ramifié, un groupe phényle, un halogène, un groupe alkoxy en C₁-C₆, NO₂, CN, SO₂NR''R''', R'' et R''' représentant indépendamment un atome d'hydrogène ou un groupe alkyle en C₁-C₆, ou un groupe CF₃, ou R⁶ et R⁷ forment ensemble un cycle supplémentaire de 4 à 7 chaînons qui peut être aromatique ou partiellement saturé, et qui peut être substitué avec un halogène, un groupe NO₂, CF₃, CN, SO₂NR''R''', R'' et R''' représentant indépendamment un atome d'hydrogène ou un groupe alkyle en C₁-C₆, et R⁴ a la même définition que ci-dessus, pour la préparation d'un médicament utile pour le traitement d'affections sensibles à un aminoacide excitant.

10. Utilisation selon la revendication 9, dans laquelle au moins l'un des groupes R⁴, R⁵, R⁶ et R⁷, représente NO₂, CF₃, CN, SO₂NR''R''' ou un halogène, et R¹, R², R⁴, R⁵, R⁶, R⁷, R'' et R''' ont autrement les mêmes définitions que dans la revendication 1.

11. Utilisation selon la revendication 9, dans laquelle R⁵ représente NO₂, F, CF₃ ou CN.
